# EUROPEAN PATENT APPLICATION

(11) **EP 2 140 798 A1**
(43) Date of publication of application: **06.01.2010**
(21) Application number: 08739344.3
(22) Date of filing: 28.03.2008
(51) Int. Cl.: A61B 1/00, A61B 1/04, A61B 5/07

(54) **CAPSULE MEDICAL DEVICE AND METHOD OF PRODUCING CAPSULE MEDICAL DEVICE**

(30) Priority: 30.03.2007 JP 2007094890; 15.10.2007 JP 2007268247
(71) Applicant: Olympus Medical Systems Corp., Tokyo 151-0072 (JP); Olympus Corporation, Tokyo 151-0072 (JP)
(72) Inventor: SEGAWA, Hidetake, Tokyo 151-0072 (JP); ORIHARA, Tatsuya, Tokyo 151-0072 (JP); FUJIMORI, Noriyuki, Tokyo 151-0072 (JP)
(74) Representative: von Hellfeld, Axel
(86) International application number: PCT/JP2008/056226
(87) International publication number: WO 2008/123465

(57) **Abstract**

An object of the present invention is to suppress variations in relative positional relation of an optical dome of a capsule casing, an illuminating unit, and an optical unit, and to determine the respective preferable relative positions of the illuminating unit and the optical unit with respect to the optical dome highly accurately. A capsule medical device according to the present invention includes optical domes 2b and 2c; illuminating boards 19a and 19f having light-emitting elements 3a to 3d and 6a to 6d mounted thereon, respectively, which illuminate inside a subject over their respective optical domes 2b and 2c; optical units 4 and 7 that form images of inside the subject for solid-state imaging devices 5 and 8; and positioning units 14 and 15 in which the illuminating boards 19a and 19f and the optical units 4 and 7 are fixedly arranged, respectively. The positioning units 14 and 15 are fitted and fixed to inner circumferences of their respective optical domes 2b and 2c, thereby determining the respective relative positions of the light-emitting elements 3a to 3d and 6a to 6d, and the optical units 4 and 7 with respect to the optical domes 2b and 2c.

## Description

### TECHNICAL FIELD

The present invention relates to a capsule medical device introduced into internal organs of a subject such as a patient to acquire in-vivo information of the subject, and a method of manufacturing a capsule medical device.

### BACKGROUND ART

Conventionally, in the field of endoscope, a swallowing-type capsule endoscope having an imaging function and a wireless communication function has been proposed. The capsule endoscope is introduced into internal organs by swallowing it from a mouth of the subject such as a patient for observation (examination) of the internal organs. Thereafter, the capsule endoscope moves in the internal organs with peristaltic movements or the like, while sequentially capturing images of inside of the subject (hereinafter, occasionally "in-vivo images") at a predetermined interval, for example, at an interval of 0.5 second, and finally, it is naturally discharged to the outside of the subject.

The in-vivo images captured by the capsule endoscope while the capsule endoscope is present inside the internal organs of the subject are sequentially transmitted from the capsule endoscope to an external receiving device by wireless communication. The receiving device is carried by the subject to receive an in-vivo image group wirelessly transmitted from the capsule endoscope introduced into the internal organs of the subject, and stores the received in-vivo image group on a recording medium.

The in-vivo image group stored on the recording medium of the receiving device is taken in an image display device such as a workstation. The image display device displays the in-vivo image group of the subject acquired via the recording medium. A doctor, a nurse or the like can diagnose the subject by observing the in-vivo image group displayed on the image display device.

Such a capsule endoscope has a capsule casing having a transparent optical dome at an end thereof and includes, inside the capsule casing, an illuminating unit such as an LED that illuminates inside of internal organs over the optical dome, an optical unit such as a lens that forms reflected light from inside of the internal organs illuminated by the illuminating unit, and an imaging unit such as a CCD that captures images of the inside of the internal organs (that is, in-vivo images) formed by the optical unit. In this case, the illuminating unit and the imaging unit are incorporated in the capsule casing, in a state of being mounted on an illuminating board and an imaging board, respectively, and the optical unit is fitted to the imaging unit on the imaging board, and incorporated in the capsule casing in a state of being inserted into a through hole formed at a center of the illuminating board (for example, see Patent Documents 1 and 2).

Patent Document 1: Japanese Patent Application Laid-open No. 2005-198964
Patent Document 2: Japanese Patent Application Laid-open No. 2005-204924

### DISCLOSURE OF INVENTION

### PROBLEM TO BE SOLVED BY THE INVENTION

Each of the illuminating board and the imaging board incorporated in the above-described conventional capsule endoscope is a rigid circuit board (hereinafter, simply "rigid board") formed in a disk-like shape so as to be arranged in the capsule casing. These boards are electrically connected with each other via a flexible circuit board (hereinafter, simply "flexible board"). The illuminating board determines relative positions of the optical dome, the illuminating unit, and the optical unit by fitting an outer edge thereof to an internal wall of the optical dome.

However, because the illuminating board described above is formed in a disk-like shape by punching of a rigid board, it generally has an external diameter tolerance of about ± 0.1 millimeter. Further, an internal diameter of the optical dome of the capsule casing described above needs to be designed to have a tolerance considering the external diameter tolerance of the illuminating board. Therefore, when the outer edge of the illuminating board having such an external diameter tolerance is fitted to the internal wall of the optical dome, the variation in relative positions of the optical dome, the illuminating unit, and the optical unit is large, thereby making it difficult to highly accurately determine preferable relative positions of the illuminating unit and the optical unit with respect to the optical dome. If the illuminating unit and the optical unit incorporated in the capsule endoscope are not arranged at preferable relative positions with respect to the optical dome, light emitted by the illuminating unit may be reflected by the optical dome and then enter into a lens of the optical unit, thereby causing flare.

The present invention has been made to solve the above problems, and an object of the present invention is to provide a capsule medical device that can suppress variations in relative positions of an optical dome of a capsule casing, an illuminating unit, and an optical unit, and can determine preferable relative positions of the illuminating unit and the optical unit with respect to the optical dome highly accurately, and a method of manufacturing a capsule medical device.

### MEANS FOR SOLVING PROBLEM

To solve the problem described above and achieve the object, a capsule medical device according to the present invention includes an optically transparent optical dome forming a part of a capsule casing introduced into a subject; an illuminating board having an illuminating unit that illuminates inside the subject over the optical dome fixedly arranged thereon; an optical unit that forms an image of inside the subject illuminated by the illuminating unit; an imaging unit that is fixedly arranged with respect to the optical unit and captures images of inside the subject; and a positioning unit in which the illuminating board and the optical unit are fixedly arranged, which is fitted and fixed to an inner circumference of the optical dome to determine relative positions of the illuminating board and the optical unit with respect to the optical dome.

In the capsule medical device according to the present invention, the positioning unit includes a plate-like portion having the illuminating board and the optical unit fixedly arranged thereon, and having an outer circumference fitted to an inner circumference of the optical dome; and a protrusion protruding from the plate-like portion for fixing the plate-like portion to the inner circumference of the optical dome by being engaged with an opening end of the optical dome.

In the capsule medical device according to the present invention, the positioning unit is a plate-like portion having the illuminating board and the optical unit fixedly arranged thereon, and having an outer circumference fitted to an inner circumference of the optical dome. The optical dome has a step for connecting a first inner circumference to be fitted to the outer circumference of the plate-like portion with a second inner circumference having an internal diameter smaller than that of the first inner circumference, so that an outer edge of the plate-like portion is engaged with the step, thereby fixing the plate-like portion to the inner circumference of the optical dome.

In the capsule medical device according to the present invention, the optical unit includes one or more lenses that form an image of inside the subject onto a light receiving surface of the imaging unit; and a lens frame that holds the one or more lenses. The lens frame is inserted into and fixed in a through hole formed in the plate-like portion.

The capsule medical device according to the present invention, the optical unit includes one or more lenses that form an image of inside the subject on a light receiving surface of the imaging unit. The plate-like portion integrally includes a lens frame that holds the one or more lenses.

In the capsule medical device according to the present invention, the lens frame includes an abutment portion protruding in a radial direction of the lens frame, which forms an external diameter larger than an internal diameter of the through hole formed in the plate-like portion. The illuminating board has an opening part into which the lens frame is inserted, and is fixedly arranged on a surface of the plate-like portion opposed to the optical dome, in a state with the lens frame being inserted into the opening part and the plate-like portion being abutted to the abutment portion.

In the capsule medical device according to the present invention, the illuminating board has an opening part into which the lens frame is inserted, and is fixedly arranged on a surface of the plate-like portion opposed to the optical dome, in a state with the lens frame being inserted into the opening part.

In the capsule medical device according to the present invention, the illuminating board is fixed by a bonding member on the surface of the plate-like portion.

In the capsule medical device according to the present invention, the illuminating board is fixed to the lens frame with an adhesive applied to a skirt of the lens frame protruding via the opening part.

In the capsule medical device according to the present invention, the plate-like portion includes one or more hooks that snap-fit the illuminating board on the surface opposed to the optical dome, and the illuminating board is fixed to the plate-like portion by snap-fitting by the one or more hooks.

The capsule medical device according to the present invention further includes a pressing unit that is screwed to a skirt of the lens frame protruding via the opening part, and presses the illuminating board to the plate-like portion. The illuminating board is fixed to the lens frame by a pressing force of the pressing unit.

In the capsule medical device according to the present invention, the positioning unit determines a relative position of the illuminating unit with respect to the optical dome in a state with an upper end of the illuminating unit being positioned at a position lower than an upper end of the lens frame.

In the capsule medical device according to the present invention, the illuminating board is a flexible circuit board.

In the capsule medical device according to the present invention, the positioning unit further determines a relative position of the imaging unit with respect to the optical dome.

The capsule medical device according to the present invention further includes an antenna that transmits a wireless signal including an image inside the subject captured by the imaging unit to outside. The positioning unit further determines a relative position of the antenna with respect to the optical dome.

The capsule medical device according to the present invention further includes an elastic member that generates a biasing force for biasing the positioning unit in a direction of pressing the positioning unit toward the optical dome; and a load receiving unit that receives the biasing force and transmits the biasing force to the positioning unit, The positioning unit is fitted and fixed to the inner circumference of the optical dome by the biasing force of the elastic member transmitted via the load receiving unit.

The capsule medical device according to the present invention further includes a power supply unit that supplies power to at least the imaging unit. The elastic member is a contact spring that ensures a contact point of the power supply unit.

In the capsule medical device according to the present invention, the optical dome has a contact surface with which at least a part of the positioning unit comes into contact.

In the capsule medical device according to the present invention, the positioning unit is a resin member formed by resin molding.

In the capsule medical device according to the present invention, the imaging unit includes a plurality of imaging units that captures images of inside the subject in a direction different from each other. The capsule casing includes a plurality of the optical domes corresponding to the plurality of the imaging units. A plurality of sets, corresponding to the plurality of the imaging units, of the illuminating unit, the illuminating board, the optical unit, the positioning unit, and the load receiving unit are incorporated in the capsule casing. The biasing force of the elastic member acts in a direction opposing to each of the imaging units.

In the capsule medical device according to the present invention, an expansion and contraction distance of the elastic member is larger than a size variation of a built-in unit of the capsule casing in an acting direction of the biasing force.

In the capsule medical device according to the present invention, the optical dome is fitted to an end of a cylindrical body forming a remaining part of the capsule casing by engagement of protruding and depressed portions. The biasing force of the elastic member is smaller than a retaining force of the optical dome and the cylindrical body by the engagement of protruding and depressed portions.

In the capsule medical device according to the present invention, an external diameter of the built-in unit of the capsule casing is smaller than an internal diameter of the cylindrical body.

The capsule medical device according to the present invention further includes an O-ring that closes a space on a connecting surface between the optical dome and the cylindrical body.

In the capsule medical device according to the present invention, the optical dome is provided with an index with a state that a relative position with respect to the built-in unit of the capsule casing can be viewed from outside.

A capsule medical device according to the present invention includes an optically transparent optical dome forming a part of a capsule casing introduced into a subject; an illuminating board having an illuminating unit that illuminates inside the subject over the optical dome fixedly arranged thereon; an optical unit that forms an image of inside the subject illuminated by the illuminating unit; an imaging unit that is fixedly arranged with respect to the optical unit and captures images of inside the subject; a positioning unit in which the illuminating board and the optical unit are fixedly arranged, which is fitted and fixed to an inner circumference of the optical dome to determine relative positions of the illuminating board and the optical unit with respect to the optical dome; an elastic member that generates a biasing force for biasing the positioning unit in a direction of pressing the positioning unit toward the optical dome; and a load receiving unit that receives the biasing force and transmits the biasing force to the positioning unit. The positioning unit is fitted and fixed to the inner circumference of the optical dome by the biasing force of the elastic member transmitted via the load receiving unit.

A capsule medical device according to the present invention includes an optically transparent optical dome forming a part of a capsule casing introduced into a subject; an illuminating board having an illuminating unit that illuminates inside the subject over the optical dome fixedly arranged thereon; an optical unit that forms an image of inside the subject illuminated by the illuminating unit; an imaging unit that is fixedly arranged with respect to the optical unit and captures images of inside the subject; a positioning unit in which the illuminating board and the optical unit are fixedly arranged, which is fitted and fixed to an inner circumference of the optical dome to determine relative positions of the illuminating board and the optical unit with respect to the optical dome; an elastic member that generates a biasing force for biasing the positioning unit in a direction of pressing the positioning unit toward the optical dome; a control unit that controls an operation of the imaging unit; and a support body that supports a circuit board having the control unit mounted thereon and ensures board spacing between the circuit board and another board. The support body is brought into contact with and fitted to the positioning unit to transmit the biasing force of the elastic member, thereby pressing the positioning unit to the optical dome.

A method of manufacturing a capsule medical device according to the present invention includes a step of fixedly arranging an optical unit for forming light on an imaging unit in the imaging unit; a step of inserting the optical unit into a through hole formed in a positioning unit to fix the optical unit therein; a step of inserting the optical unit into a through hole formed in an illuminating board having an illuminating unit to fix the optical unit in the positioning unit; and a step of determining respective relative positions of the optical unit, the illuminating board, and the optical dome by fitting and fixing the positioning unit to an inner circumference of the optical dome forming a part of a capsule casing.

The method of manufacturing a capsule medical device according to the present invention further includes a step of maintaining the respective relative positions of the optical unit, the illuminating board, and the optical dome by pressing the positioning unit to the optical dome by a biasing force generated by an elastic member.

### EFFECT OF THE INVENTION

In the capsule medical device according to the present invention, an illuminating board having an illuminating unit that illuminates inside of a subject over an optical dome fixedly arranged thereon and an optical unit that forms an image of inside the illuminated subject on a light receiving surface of an imaging unit are fixedly arranged in a positioning unit, to determine relative positions of the illuminating unit and the optical unit with respect to the optical dome by fitting and fixing an outer circumference of the positioning unit to an inner circumference of the optical dome. Therefore, variations in relative positions of the optical dome, the illuminating unit, and the optical unit can be suppressed, and preferable relative positions of the illuminating unit and the optical unit with respect to the optical dome can be determined highly accurately.

### BRIEF DESCRIPTION OF DRAWINGS

FIG. 1 is a schematic longitudinal cross section of a configuration example of a capsule endoscope according to an embodiment of the present invention.
FIG. 2 is a schematic diagram for exemplifying an internal structure of the capsule endoscope as viewed over an optical dome from a direction F shown in FIG. 1.
FIG. 3 is a schematic diagram for exemplifying the internal structure of the capsule endoscope as viewed over the optical dome from a direction B shown in FIG. 1.
FIG. 4 is a schematic diagram for exemplifying a state where circuit components of a power supply system are mounted on a control board.
FIG. 5 is a schematic diagram for exemplifying a state where a series of circuit boards folded and arranged in a casing of the capsule endoscope is developed.
Fig. 6 is a schematic diagram for explaining a positioning effect of a light-emitting element and an optical unit with respect to optical domes.
Fig. 7 is a schematic diagram for exemplifying an engaging state of an opening end of an optical dome and a load receiving unit with respect to a flange of a positioning unit.
Fig. 8 is a schematic longitudinal cross section of a configuration example of a capsule endoscope according to a first modification of the first embodiment of the present invention.
Fig. 9 is a schematic longitudinal cross section of a configuration example of a capsule endoscope according to a second modification of the first embodiment of the present invention.
Fig. 10 is a schematic longitudinal cross section of a configuration example of a capsule endoscope according to a second embodiment of the present invention.
Fig. 11 is a schematic diagram for exemplifying a state where an index formed on an optical dome is viewed from outside.
Fig. 12 is a schematic longitudinal cross section of a configuration example of a capsule endoscope according to a third embodiment of the present invention.
Fig. 13 is a schematic longitudinal cross section for exemplifying a part of an optical dome where an O-ring is arranged.
Fig. 14 is a schematic cross section for exemplifying a state where an illuminating board is fixed to a positioning unit by an adhesive applied to a skirt of a lens frame.
Fig. 15 is a schematic cross section for exemplifying a state where the illuminating board is snap-fitted to the positioning unit by a hook.
Fig. 16 is a schematic cross section for exemplifying a state where the illuminating board is pressed and fixed to the positioning unit by a pressing member screwed to a lens frame.
Fig. 17 is a schematic cross section of a configuration example of a monocular capsule medical device according to the present invention.

### EXPLANATIONS OF LETTERS OR NUMERALS

- 1, 21, 31, 41, 51, 61: Capsule endoscope
- 2, 32, 42, 52, 62: Casing
- 2a, 62a: Cylindrical body
- 2b, 2c, 32b, 32c, 42b, 42c, 52b, 52c: Optical dome
- 3a to 3d, 6a to 6d: Light-emitting element
- 4, 7: Optical unit
- 4a, 4b, 7a, 7b: Lens
- 4c, 7c: Aperture unit
- 4d, 7d: Lens frame
- 5, 8: Solid-state imaging device
- 9a: Wireless unit
- 9b: Antenna
- 10: Control unit
- 11a: Magnetic switch
- 11b, 11c: Capacitor
- 11d: Power supply IC
- 12a, 12b: Battery
- 13a, 13b: Contact spring
- 14, 15, 24, 25, 34, 35: Positioning unit
- 14a, 15a, 24a, 25a: Plate-like portion
- 14b, 15b: Protrusion
- 14c: Engaging unit
- 14d: Flange
- 16, 17, 36: Load receiving unit
- 18a, 18b: Power supply board
- 19a, 19f: Illuminating board
- 19b, 19e: Imaging board
- 19c: Control board
- 19d: Wireless board
- 20: Series of circuit boards
- 20a, 20b: Series of flexible boards
- 53a, 53b: O-ring
- 62c: Step
- 71: Adhesive
- 72: Hook
- 73: Pressing unit
- A1 to A5: Extending part
- CL: Central axis
- M1, M2: Index
- H1, H2: Opening part
- TP1, TP2: Top part

### BEST MODE(S) FOR CARRYING OUT THE INVENTION

Exemplary embodiments of a capsule medical device and a method of manufacturing a capsule medical device according to the present invention will be explained below in detail with reference to the accompanying drawings. A capsule endoscope introduced into a subject and having an imaging function for capturing an in-vivo image, which is an example of in-vivo information of the subject, and a wireless communication function for wirelessly transmitting the captured in-vivo image is explained as an example of the capsule medical device of the present invention. However, the present invention is not limited to the embodiments.

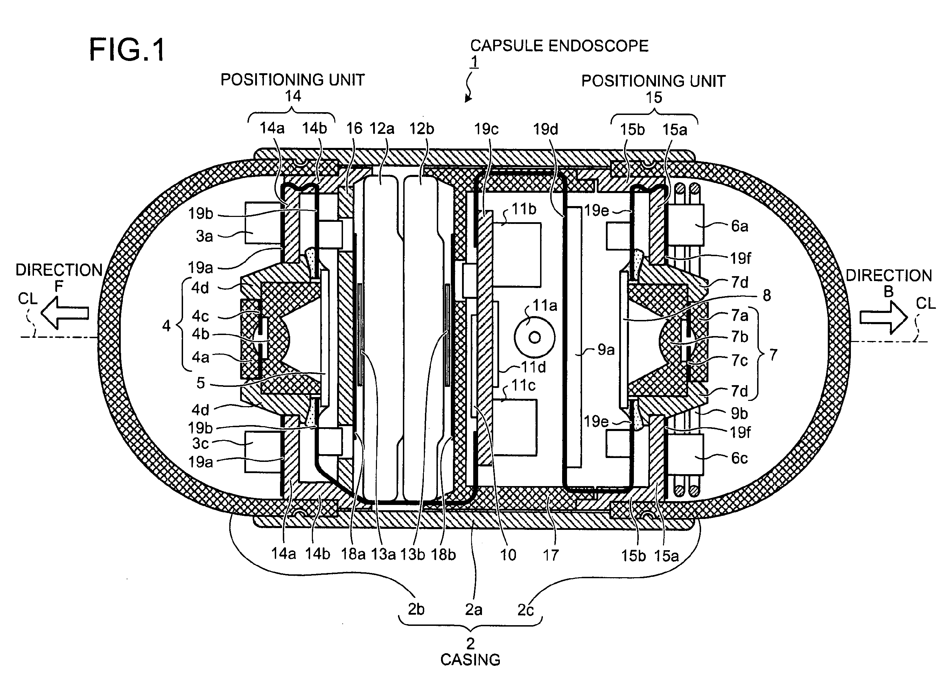

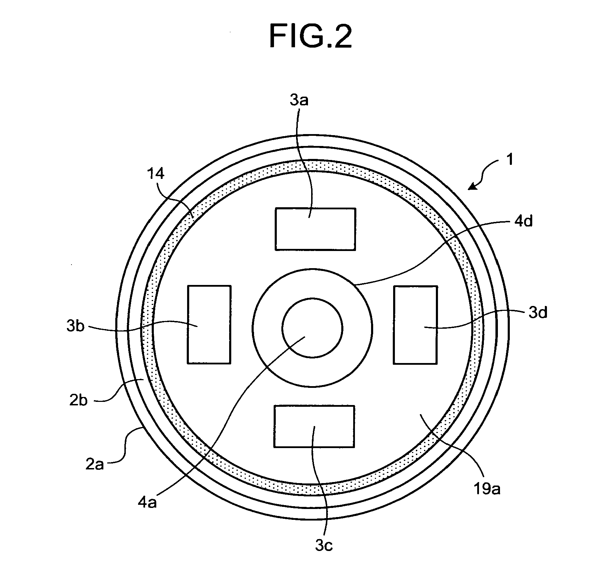

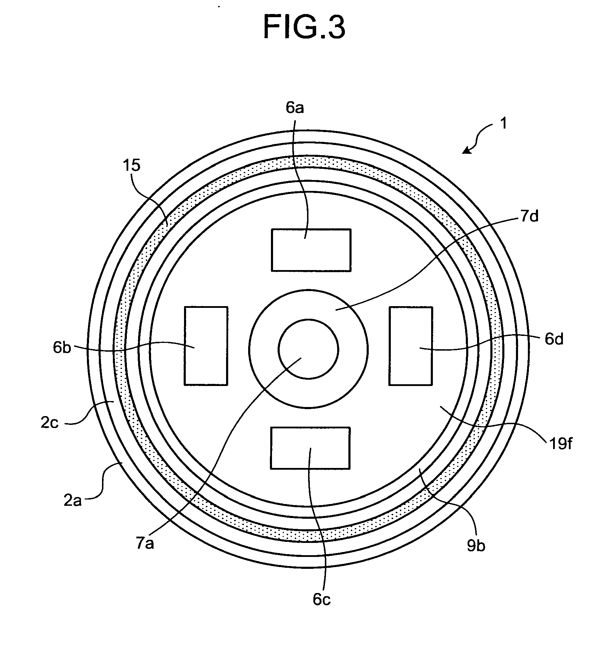

(First Embodiment) FIG. 1 is a longitudinal cross section of a configuration example of a capsule endoscope according to a first embodiment of the present invention. FIG. 2 is a schematic diagram for exemplifying an internal structure of the capsule endoscope as viewed over an optical dome from a direction F shown in FIG. 1. FIG. 3 is a schematic diagram for exemplifying the internal structure of the capsule endoscope as viewed over the optical dome from a direction B shown in FIG. 1.

As shown in FIG. 1, a capsule endoscope 1 according to the embodiment of the present invention is a binocular-lens capsule endoscope that captures an in-vivo image on a direction F side (forward side) and an in-vivo image on a direction B side (back side). The capsule endoscope 1 includes a capsule casing 2 formed in a size introduceable into internal organs of a subject, and has an imaging function for capturing an in-vivo image on the direction F side, an imaging function for capturing an in-vivo image on the direction B side, and a wireless communication function for wirelessly transmitting in-vivo images captured by these imaging functions to the outside.

Specifically, as shown in FIGS. 1 to 3, the capsule endoscope 1 includes, in the casing 2, an illuminating board 19a including a plurality of light-emitting elements 3a to 3d mounted thereon to illuminate the inside of the subject on the direction F side; an optical unit 4 that forms images of inside the subject illuminated by the light-emitting elements 3a to 3d; and an imaging board 19b including a solid-state imaging device 5 mounted thereon to capture the images of inside the subject formed by the optical unit 4 (that is, the in-vivo image on the direction F side). The capsule endoscope 1 also includes, in the casing 2, an illuminating board 19f including a plurality of light-emitting elements 6a to 6d mounted thereon to illuminate the inside of the subject on the direction B side; an optical unit 7 that forms images of inside the subject illuminated by the light-emitting elements 6a to 6d; and an imaging board 19e including a solid-state imaging device 8 mounted thereon to capture the images of inside the subject formed by the optical unit 7 (that is, the in-vivo image on the direction B side). Further, the capsule endoscope 1 includes, in the casing 2, a wireless board 19d having a wireless unit 9a mounted thereon to wirelessly transmit respective in-vivo images captured by the solid-state imaging devices 5 and 8 to the outside via an antenna 9b, and a control board 19c having a control unit 10 mounted thereon to control the imaging function and the wireless communication function.

The capsule endoscope 1 includes, in the casing 2, a power supply system for supplying electric power to the light-emitting elements 3a to 3d and 6a to 6d, the solid-state imaging devices 5 and 8, the wireless unit 9a, and the control unit 10, that is, various circuit parts such as a magnetic switch 11a; batteries 12a and 12b; power supply boards 18a and 18b; and contact springs 13a and 13b that connect the batteries 12a and 12b with the power supply boards 18a and 18b so that electrical conduction therebetween is established. Further, the capsule endoscope 1 also includes, in the casing 2, a positioning unit 14 that determines respective relative positions of the light-emitting elements 3a to 3d and the optical unit 4 with respect to an optical dome 2b forming the forward end of the casing 2; a positioning unit 15 that determines respective relative positions of the light-emitting elements 6a to 6d and the optical unit 7 with respect to an optical dome 2c forming the backward end of the casing 2; a load receiving unit 16 that receives an elastic force of the contact spring 13a to fix the positioning unit 14 with respect to the optical dome 2b; and a load receiving unit 17 that receives an elastic force of the contact spring 13b to fix the positioning unit 15 with respect to the optical dome 2c.

The casing 2 is a capsule casing having a size easily introduceable into the internal organs of the subject, and is realized by fitting the optical domes 2b and 2c to both opening end portions of a cylindrical body 2a having a cylindrical structure. The cylindrical body 2a has an outer diameter larger than that of the optical domes 2b and 2c, so that the optical domes 2b and 2c can be fitted to an inner circumference near the both opening ends. A step that abuts against the end face of the optical domes 2b and 2c at the time of fitting the optical domes 2b and 2c is formed on the inner circumference near the both opening ends of the cylindrical body 2a. The relative positions of the optical domes 2b and 2c with respect to the cylindrical body 2a are determined by abutting the respective end faces of the optical domes 2b and 2c against the step of the cylindrical body 2a.

The optical domes 2b and 2c are optically transparent dome members formed in a substantially uniform thickness. A depression is formed on an outer circumference near the opening end of each of the optical domes 2b and 2c. The depressions engage with protrusions provided on the inner circumference near the opening ends of the cylindrical body 2a. The optical dome 2b is fitted to the inner circumference near the opening end on the forward side (the direction F side shown in FIG. 1) of the cylindrical body 2a, and is attached to the forward-side opening end of the cylindrical body 2a by locking the protrusion on the inner circumference of the cylindrical' body 2a in the depression of the optical dome 2b. In this case, the end face of the optical dome 2b is in a state of being abutted against the step on the inner circumference of the cylindrical body 2a. The optical dome 2b forms a part of the capsule casing 2 (specifically, a forward end). Meanwhile, the optical domes 2c is fitted to the inner circumference near the opening end on the back side (the direction B side shown in FIG. 1) of the cylindrical body 2a, and is attached to the back-side opening end of the cylindrical body 2a by locking the protrusion on the inner circumference of the cylindrical body 2a in the depression of the optical dome 2c. In this case, the end face of the optical dome 2c is in a state of being abutted against the step on the inner circumference of the cylindrical body 2a. The optical dome 2c forms a part of the capsule casing 2 (specifically, a backward end). As shown in FIG. 1, the casing 2 including the cylindrical body 2a and the optical domes 2b and 2c liquid-tightly accommodates the respective components of the capsule endoscope 1.

The light-emitting elements 3a to 3d function as an illuminating unit that illuminates the inside of the subject positioned on the direction F side. Specifically, each of the light-emitting elements 3a to 3d is a light-emitting element such as an LED, and is mounted on the illuminating board 19a, which is a flexible board formed in a substantially disk shape. In this case, as shown in FIGS. 1 and 2, the light-emitting elements 3a to 3d are mounted on the illuminating board 19a to surround a lens frame 4d (described later) of the optical unit 4 inserted into an opening part of the illuminating board 19a. The light-emitting elements 3a to 3d emit predetermined illumination light (for example, white light), to illuminate the inside of the subject on the direction F side over the forward-side optical dome 2b.

The number of the light-emitting elements to be mounted on the illuminating board 19a is not specifically limited to four, and can be one or more, so long as the light-emitting element can emit the illumination light with an amount of light sufficient for illuminating the inside of the subject on the direction F side. As exemplified in the light-emitting elements 3a to 3d, when a plurality of light-emitting elements are mounted on the illuminating board 19a, it is desired that the light-emitting elements are mounted thereon at rotationally symmetric positions centering on an optical axis of the optical unit 4 inserted into the opening part of the illuminating board 19a.

The optical unit 4 condenses reflected light from the inside of the subject on the direction F side illuminated by the light-emitting elements 3a to 3d, and forms images of inside the subject on the direction F side. The optical unit 4 is realized by lenses 4a and 4b formed by, for example, injection molding of glass or plastic, an aperture unit 4c arranged between the lenses 4a and 4b, and the lens frame 4d that holds the lenses 4a and 4b and the aperture unit 4c.

The lenses 4a and 4b condense the reflected light from the inside of the subject on the direction F side illuminated by the light-emitting elements 3a to 3d, and forms images of inside the subject on the direction F side on a light receiving surface of the solid-state imaging device 5. The aperture unit 4c narrows down (adjusts) brightness of the reflected light condensed by the lenses 4a and 4b to suitable brightness. The lens frame 4d has a cylindrical structure with the both ends being opened, and holds the lenses 4a and 4b and the aperture unit 4c in a cylindrical portion. The lens frame 4d is fitted and fixed to a through hole in a plate-like portion 14a (described later) of the positioning unit 14, with the lens frame 4d being inserted into an opening part formed in the illuminating board 19a. In this case, an upper end (an opening end on the lens 4a side) and a body of the lens frame 4d are protruded on the illuminating board 19a side, and a lower end thereof is locked to a peripheral portion of the through hole in the plate-like portion 14a. The lens frame 4d fixed to the plate-like portion 14a of the positioning unit 14 holds the lenses 4a and 4b at predetermined positions determined by the positioning unit 14 (that is, suitable relative positions with respect to the optical dome 2b). The lenses 4a and 4b can match a longitudinal central axis CL of the casing 2 with the optical axis.

The lens 4b held by the lens frame 4d has legs as shown in FIG. 1, and determines positional relation between the lens 4b and the solid-state imaging device 5 in an optical axis direction by abutting the legs against a device surface on a light receiving side of the solid-state imaging device 5. Thus, in a manner in which the legs of the lens 4b abut against the device surface on the light receiving side of the solid-state imaging device 5, a clearance is formed between the lower end of the lens frame 4d and the imaging board 19b. A predetermined adhesive is filled in the clearance, and the lower end of the lens frame 4d and the imaging board 19b are bonded to each other by the adhesive. The adhesive and the lens frame 4d block unnecessary light from entering into the lenses 4a and 4b and the light receiving surface of the solid-state imaging device 5.

The solid-state imaging device 5 is a CCD, CMOS, or the like having the light receiving surface, and functions as an imaging unit that captures images of inside the subject on the direction F side illuminated by the light-emitting elements 3a to 3d. Specifically, the solid-state imaging device 5 is mounted (for example, flip-chip mounted) on the imaging board 19b, which is the flexible board formed in a substantially disk shape, so that the lens 4b faces the light receiving surface via an opening part of the imaging board 19b. In this case, the solid-state imaging device 5 causes the device surface thereof on the light receiving side to abut against the legs of the lens 4b, and is fixed and arranged with respect to the optical unit 4 by adhesion between the imaging board 19b and the lower end of the lens frame 4d, while maintaining the abutting state with respect to the legs of the lens 4b. The solid-state imaging device 5 receives the reflected light from the inside of the subject condensed by the lenses 4a and 4b via the light receiving surface, and captures images of inside the subject formed on the light receiving surface by the lenses 4a and 4b (that is, an in-vivo image on the direction F side).

The light-emitting elements 6a to 6d function as an illuminating unit that illuminates the inside of the subject positioned on the direction B side. Specifically, each of the light-emitting elements 6a to 6d is a light-emitting element such as an LED, and is mounted on the illuminating board 19f, which is a flexible board formed in a substantially disk shape. In this case, as shown in FIGS. 1 and 3, the light-emitting elements 6a to 6d are mounted on the illuminating board 19f to surround a lens frame 7d (described later) of the optical unit 7 inserted into an opening part of the illuminating board 19f. The light-emitting elements 6a to 6d emit predetermined illumination light (for example, white light), to illuminate the inside of the subject on the direction B side over the back-side optical dome 2c.

The number of the light-emitting elements to be mounted on the illuminating board 19f is not specifically limited to four, and can be one or more, so long as the light-emitting element can emit the illumination light with an amount of light sufficient for illuminating the inside of the subject on the direction B side. As exemplified in the light-emitting elements 6a to 6d, when a plurality of light-emitting elements are mounted on the illuminating board 19f, it is desired that the light-emitting elements are mounted thereon at rotationally symmetric positions centering on an optical axis of the optical unit 7 inserted into the opening part of the illuminating board 19f.

The optical unit 7 condenses the reflected light from the inside of the subject on the direction B side illuminated by the light-emitting elements 6a to 6d and forms images of inside the subject on the direction B side. The optical unit 7 is realized by lenses 7a and 7b formed by, for example, injection molding of glass or plastic, an aperture unit 7c arranged between the lenses 7a and 7b, and the lens frame 7d that holds the lenses 7a and 7b and the aperture unit 7c.

The lenses 7a and 7b condense the reflected light from the inside of the subject on the direction B side illuminated by the light-emitting elements 6a to 6d, and forms the images of inside the subject on the direction B side on a light receiving surface of the solid-state imaging device 8. The aperture unit 7c narrows down (adjusts) brightness of the reflected light condensed by the lenses 7a and 7b to suitable brightness. The lens frame 7d has a cylindrical structure with the both ends being opened, and holds the lenses 7a and 7b and the aperture unit 7c in a cylindrical portion. The lens frame 7d is fitted and fixed to a through hole in a plate-like portion 15a (described later) of the positioning unit 15, with the lens frame 7d being inserted into the opening part formed in the illuminating board 19f. In this case, an upper end (an opening end on the lens 7a side) and a body of the lens frame 7d are protruded on the illuminating board 19f side, and a lower end thereof is locked to a peripheral portion of the through hole in the plate-like portion 15a. The lens frame 7d fixed to the plate-like portion 15a of the positioning unit 15 holds the lenses 7a and 7b at predetermined positions determined by the positioning unit 15 (that is, suitable relative positions with respect to the optical dome 2c). The lenses 7a and 7b can match the longitudinal central axis CL of the casing 2 with the optical axis.

The lens 7b held by the lens frame 7d has legs (see FIG. 1) as the lens 4b of the optical unit 4, and determines positional relation between the lens 7b and the solid-state imaging device 8 in the optical axis direction by abutting the legs against a device surface on a light receiving side of the solid-state imaging device 8. Thus, in a manner in which the legs of the lens 7b abut against the device surface on the light receiving side of the solid-state imaging device 8, a clearance is formed between the lower end of the lens frame 7d and the imaging board 19e. A predetermined adhesive is filled in the clearance, and the lower end of the lens frame 7d and the imaging board 19e are bonded to each other by the adhesive. The adhesive and the lens frame 7d block unnecessary light from entering into the lenses 7a and 7b and the light receiving surface of the solid-state imaging device 8.

The solid-state imaging device 8 is a CCD, CMOS, or the like having the light receiving surface, and functions as an imaging unit that captures images of inside the subject on the direction B side illuminated by the light-emitting elements 6a to 6d. Specifically, the solid-state imaging device 8 is mounted (for example, flip-chip mounted) on the imaging board 19e, which is a flexible board formed in a substantially disk shape, so that the lens 7b faces the light receiving surface via an opening part of the imaging board 19e. In this case, the solid-state imaging device 8 causes the device surface thereof on the light receiving side to abut against the legs of the lens 7b, and is fixed and arranged with respect to the optical unit 7 by adhesion between the imaging board 19e and the lower end of the lens frame 7d, while maintaining the abutting state with respect to the legs of the lens 7b. The solid-state imaging device 8 receives the reflected light from the inside of the subject condensed by the lenses 7a and 7b via the light receiving surface, and captures images of inside the subject formed on the light receiving surface by the lenses 7a and 7b (that is, an in-vivo image on the direction B side).

The wireless unit 9a and the antenna 9b realize the wireless communication function for wirelessly transmitting each of in-vivo images on the direction F or the direction B side captured by the solid-state imaging devices 5 and 8 to the outside. Specifically, the wireless unit 9a is mounted on the wireless board 19d, which is the flexible board formed in a substantially disk shape, and is arranged in the casing 2, facing the imaging board 19e having the solid-state imaging device 8 mounted thereon. As shown in FIGS. 1 and 3, the antenna 9b is fixed and arranged on the illuminating board 19f fixed on the surface of the plate-like portion 15a of the positioning unit 15, and is connected to the wireless unit 9a via the wireless board 19d and the illuminating board 19f. In this case, the antenna 9b is fixed and arranged on an outer edge of the illuminating board 19f facing the optical dome 2c at the backward end and outside of the light-emitting elements 6a to 6d.

When having acquired an image signal including the in-vivo image on the direction F side captured by the solid-state imaging device 5, the wireless unit 9a performs modulation or the like with respect to the acquired image signal each time, to generate a wireless signal including the in-vivo image on the direction F side, and transmits the generated wireless signal to the outside via the antenna 9b. Meanwhile, when having acquired an image signal including the in-vivo image on the direction B side captured by the solid-state imaging device 8, the wireless unit 9a performs modulation or the like with respect to the acquired image signal each time, to generate a wireless signal including the in-vivo image on the direction B side, and transmits the generated wireless signal to the outside via the antenna 9b. The wireless unit 9a alternately generates the wireless signal including the in-vivo image on the direction F side and the wireless signal including the in-vivo image on the direction B side under control of the control unit 10, and alternately transmits the generated wireless signals.

The control unit 10 is a processor such as a DSP, and is arranged approximately at the center of the casing 2 in a state mounted on the control board 19c, which is a rigid board formed in a substantially disk shape. The control unit 10 is electrically connected to the illuminating boards 19a and 19f, the imaging boards 19b and 19e, and the wireless board 19d via the control board 19c and the flexible board. The control unit 10 controls: the light-emitting elements 3a to 3d mounted on the illuminating board 19a; the light-emitting elements 6a to 6d mounted on the illuminating board 19f; the solid-state imaging devices 5 and 8 mounted on the imaging boards 19b and 19e, respectively; and the wireless unit 9a mounted on the wireless board 19d. Specifically, the control unit 10 controls operation timing of the light-emitting elements 3a to 3d and the solid-state imaging device 5 so that the solid-state imaging device 5 captures the in-vivo image on the direction F side for each predetermined time period, synchronously with a light emitting operation of the light-emitting elements 3a to 3d. Likewise, the control unit 10 controls the operation timing of the light-emitting elements 6a to 6d and the solid-state imaging device 8 so that the solid-state imaging device 8 captures the in-vivo image on the direction B side for each predetermined time period, synchronously with the light emitting operation of the light-emitting elements 6a to 6d. The control unit 10 also controls the wireless unit 9a to wirelessly transmit the in-vivo image on the direction F side and the in-vivo image on the direction B side alternately. The control unit 10 includes various parameters involved with image processing such as white balance, and has an image processing function for sequentially generating the image signal including the in-vivo image on the direction F side captured by the solid-state imaging device 5 and the image signal including the in-vivo image on the direction B side captured by the solid-state imaging device 8.

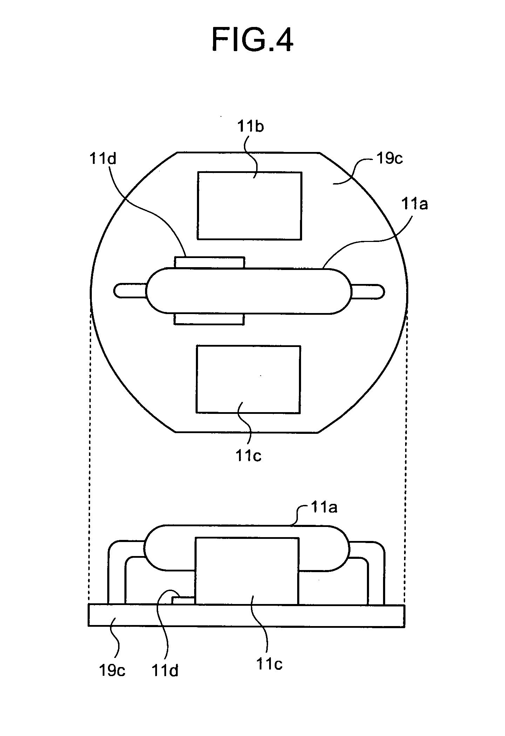

Meanwhile, on the control board 19c, circuit components of the power supply system, that is, various circuit components such as the magnetic switch 11a are mounted on a board surface on the opposite side of the board surface where the control unit 10 is mounted. FIG. 4 is a schematic diagram for exemplifying a state where the circuit components of the power supply system are mounted on the control board 19c. As shown in FIGS. 1 and 4, for example, the magnetic switch 11a, capacitors 11b and 11c, and a power supply IC 11d are mounted on one board surface of the control board 19c, as the circuit components of the power supply system. In this case, the capacitors 11b and 11c and the power supply IC 11d are surface-mounted on the control board 19c, and the magnetic switch 11a is mounted on the control board 19c, spanning over the power supply IC 11d using a lead extending from the both ends of the magnetic switch 11a. The magnetic switch 11a switches ON/OFF by applying an external magnetic field in a predetermined direction. In a case of ON state, the magnetic switch 11a starts to supply power to the light-emitting elements 3a to 3d and 6a to 6d, the solid-state imaging devices 5 and 8, the wireless unit 9a, and the control unit 10 from the batteries 12a and 12b, and in a case of OFF state, the magnetic switch 11a stops supplying power from the batteries 12a and 12b. Meanwhile, the power supply IC 11d has a power supply control function for controlling the power supply to the respective components via the magnetic switch 11a.

The batteries 12a and 12b generate power for operating the light-emitting elements 3a to 3d and 6a to 6d, the solid-state imaging devices 5 and 8, the wireless unit 9a, and the control unit 10. Specifically, the batteries 12a and 12b are button batteries such as a silver oxide battery, and as shown in FIG. 1, are arranged between the load receiving units 16 and 17 and held by an end of the positioning unit 14 and an end of the load receiving unit 17. The power supply boards 18a and 18b electrically connected to the control board 19c via the flexible board or the like are provided on surfaces of the load receiving units 16 and 17, respectively, which are facing the batteries 12a and 12b, respectively. The conductive contact springs 13a and 13b are provided on the power supply boards 18a and 18b, respectively. The batteries 12a and 12b arranged between the load receiving units 16 and 17 are held by the end of the positioning unit 14 and the end of the load receiving unit 17 in a manner in which the contact springs 13a and 13b are contracted, and are electrically connected to the circuit components (the magnetic switch 11a, the capacitors 11b and 11c, and the power supply IC 11d) of the power supply system on the control board 19c via the contracted contact springs 13a and 13b and the power supply boards 18a and 18b. The number of batteries arranged in the casing 2 is not particularly limited two, so long as the required power can be supplied.

The positioning unit 14 is a resin member formed by injection molding of resin or the like. The illuminating board 19a including the light-emitting elements 3a to 3d mounted thereon and the optical unit 4 are fixed and arranged in the positioning unit 14, and the positioning unit 14 is fitted and fixed to an inner circumference of the forward-side optical dome 2b. The positioning unit 14 fitted and fixed to the inner circumference of the optical dome 2b fixes the positional relation of the optical dome 2b, the light-emitting elements 3a to 3d, and the optical unit 4, and determines suitable relative positions of the light-emitting elements 3a to 3d and the optical unit 4 with respect to the optical dome 2b. The positioning unit 14 includes the plate-like portion 14a fitted to the inner circumference of the optical dome 2b and a protrusion 14b for fixing the plate-like portion 14a at a predetermined position on the inner circumference of the optical dome 2b.

The plate-like portion 14a is a substantially disk plate member having an outer diameter matched with an inner diameter of the optical dome 2b, and has an outer circumference fitted to the inner circumference of the optical dome 2b. The illuminating board 19a and the optical unit 4 are fixed and arranged on the plate-like portion 14a. Specifically, the external diameter of the plate-like portion 14a is designed to be slightly smaller than the internal diameter of the optical dome 2b, to generate an appropriate clearance between the outer circumference of the plate-like portion 14a and the inner circumference of the optical dome 2b. The plate-like portion 14a is slidably fitted to the inner circumference of the optical dome 2b. Specifically, the plate-like portion 14a fixes and arranges the illuminating board 19a on a surface facing the optical dome 2b, when being fitted to the inner circumference of the optical dome 2b. The plate-like portion 14a has a through hole that communicates with an opening part formed in the illuminating board 19a substantially at a center thereof, and the lens frame 4d of the optical unit 4 is inserted into and fixed (for example, fitted and fixed) in the through hole. The lens frame 4d inserted into and fixed in the through hole of the plate-like portion 14a protrudes the upper end and the body thereof on the illuminating board 19a side in a state of being inserted into the opening part of the illuminating board 19a. The plate-like portion 14a fixes the positional relation between the lens frame 4d and the light-emitting elements 3a to 3d so that the respective upper ends of the light-emitting elements 3a to 3d are positioned at a lower position than the upper end of the lens frame 4d.

The protrusion 14b protrudes from the plate-like portion 14a, and is locked to the opening end of the optical dome 2b to fix the plate-like portion 14a on the inner circumference of the optical dome 2b. Specifically, the protrusion 14b is integrally formed with the plate-like portion 14a, and protrudes from a back of the surface of the plate-like portion 14a, on which the illuminating board 19a is fixed and arranged. The protrusion 14b has a cylindrical structure having an external diameter matched with the internal diameter of the optical dome 2b (that is, an external diameter equal to that of the plate-like portion 14a), to generate an appropriate clearance between the outer circumference of the cylindrical structure and the inner circumference of the optical dome 2b. The protrusion 14b has a flange engaged with the opening end of the optical dome 2b at the opening end of the cylindrical structure. The protrusion 14b having such a structure engages the flange at the opening end of the optical dome 2b by the elastic force of the contact spring 13a, while being slidably fitted to the inner circumference of the optical dome 2b together with the plate-like portion 14a, thereby fixing the plate-like portion 14a at a predetermined position on the inner circumference of the optical dome 2b. An external diameter of the flange of the protrusion 14b is designed to be slightly smaller than the internal diameter of the cylindrical body 2a, to generate an appropriate clearance between the outer circumference of the flange of the protrusion 14b and the inner circumference of the cylindrical body 2a.

The positioning unit 15 is a resin member formed by injection molding of resin or the like. The illuminating board 19f including the light-emitting elements 6a to 6d mounted thereon and the optical unit 4 are fixed and arranged in the positioning unit 15, and the positioning unit 15 is fitted and fixed to an inner circumference of the backward-side optical dome 2c. The positioning unit 15 fitted and fixed to the inner circumference of the optical dome 2c fixes the positional relation of the optical dome 2c, the light-emitting elements 6a to 6d, and the optical unit 7, and determines suitable relative positions of the light-emitting elements 6a to 6d and the optical unit 7 with respect to the optical dome 2c. The positioning unit 15 includes the plate-like portion 15a fitted to the inner circumference of the optical dome 2c and a protrusion 15b for fixing the plate-like portion 15a at a predetermined position on the inner circumference of the optical dome 2c.

The plate-like portion 15a is a substantially disk plate member having an outer diameter matched with an inner diameter of the optical dome 2c, and has an outer circumference fitted to the inner circumference of the optical dome 2c. The illuminating board 19f and the optical unit 7 are fixed and arranged on the plate-like portion 15a. Specifically, the external diameter of the plate-like portion 15a is designed to be slightly smaller than the internal diameter of the optical dome 2c, to generate an appropriate clearance between the outer circumference of the plate-like portion 15a and the inner circumference of the optical dome 2c. The plate-like portion 15a is slidably fitted to the inner circumference of the optical dome 2c. Specifically, the plate-like portion 15a fixes and arranges the illuminating board 19f on a surface facing the optical dome 2c, when being fitted to the inner circumference of the optical dome 2c. The plate-like portion 15a has a through hole that communicates with an opening part formed in the illuminating board 19f substantially at a center thereof, and the lens frame 7d of the optical unit 7 is inserted into and fixed (for example, fitted and fixed) in the through hole. The lens frame 7d inserted into and fixed in the through hole of the plate-like portion 15a protrudes the upper end and the body thereof on the illuminating board 19f side in a state of being inserted into the opening part of the illuminating board 19f. The plate-like portion 15a fixes the positional relation between the lens frame 7d and the light-emitting elements 6a to 6d so that the respective upper ends of the light-emitting elements 6a to 6d are positioned at a lower position than the upper end of the lens frame 7d.

The protrusion 15b protrudes from the plate-like portion 15a, and is locked to the opening end of the optical dome 2c to fix the plate-like portion 15a on the inner circumference of the optical dome 2c. The protrusion 15b has a cylindrical structure having an external diameter matched with the internal diameter of the optical dome 2c (that is, an external diameter equal to that of the plate-like portion 15a), to generate an appropriate clearance between the outer circumference of the cylindrical structure and the inner circumference of the optical dome 2c. The protrusion 15b has a flange engaged with the opening end of the optical dome 2c at the opening end of the cylindrical structure. The protrusion 15b having such a structure engages the flange at the opening end of the optical dome 2c by the elastic force of the contact spring 13b, while being slidably fitted to the inner circumference of the optical dome 2c together with the plate-like portion 15a, thereby fixing the plate-like portion 15a at a predetermined position on the inner circumference of the optical dome 2c. An external diameter of the flange of the protrusion 15b is designed to be slightly smaller than the internal diameter of the cylindrical body 2a, to generate an appropriate clearance between the outer circumference of the flange of the protrusion 15b and the inner circumference of the cylindrical body 2a.

Upon reception of the elastic force (spring force) of the contact spring 13a, the load receiving unit 16 functions as a pressing unit that presses and fixes the positioning unit 15 to the opening end of the optical dome 2c by the elastic force. Specifically, the load receiving unit 16 is a plate member having a substantially disk shape that engages the outer edge thereof with a step formed on an inner circumference of the protrusion 15b of the positioning unit 14 (an engaging unit 14c to be described later), and includes the power supply board 18a and the contact spring 13a on the surface facing the battery 12a. The load receiving unit 16 presses and fixes the flange of the protrusion 14b to the opening end of the optical dome 2b by the elastic force of the contact spring 13a, upon reception of the elastic force (that is, biasing force that acts in a direction of pressing the positioning unit 14 to the inner circumference of the optical dome 2b) of the contact spring 13a generated with contraction of the contact spring 13a. In this case, the load receiving unit 16 fits and fixes the plate-like portion 14a integral with the protrusion 14b at the predetermined position on the inner circumference of the optical dome 2b by pressing and fixing the protrusion 14b to the opening end of the optical dome 2b.

As shown in FIG. 1, the through hole for avoiding a contact with the circuit components such as the capacitor mounted on the imaging board 19b is provided in the load receiving unit 16. When the load receiving unit 16 is engaged with the step on the inner circumference of the protrusion 14b, the load receiving unit 16 and the positioning unit 14 form a space, as shown in FIG. 1, sufficient for arranging the solid-state imaging device 5 abutting against the legs of the lens 4b and the imaging board 19b fixed with respect to the lower part of the lens frame 4d.

Upon reception of the elastic force (spring force) of the contact spring 13b, the load receiving unit 17 functions as a pressing unit that presses and fixes the positioning unit 15 to the opening end of the optical dome 2c by the elastic force. Specifically, the load receiving unit 17 is a member having a cylindrical structure having a slightly smaller outer diameter than an inner diameter of the cylindrical body 2a of the casing 2, and including a plate-like portion facing the battery 12b at one opening end of the cylindrical structure. The load receiving unit 17 forms an appropriate clearance between the outer circumference thereof and the inner circumference of the cylindrical body 2a.

The cylindrical structure of the load receiving unit 17 functions as a spacer that forms a predetermined space in the casing 2, and engages the other opening end with the opening end (flange) of the protrusion 15b of the positioning unit 15. In this case, as shown in FIG. 1, the cylindrical structure of the load receiving unit 17 and the positioning unit 15 forms a space sufficient for arranging the control board 19c including the control unit 10 and the circuit components such as the magnetic switch 11a mounted thereon, the wireless board 19d including the wireless unit 9a mounted thereon, the solid-state imaging device 8 abutting against the legs of the lens 7b, and the imaging board 19e fixed with respect to the lower part of the lens frame 7d. Further, the cylindrical structure of the load receiving unit 17 supports the control board 19c and the wireless board 19d in the space formed therein. That is, the load receiving unit 17 having such a cylindrical structure functions as a pressing unit that presses the positioning unit 15, and also functions as a support body that supports the control board 19c and the wireless board 19d in the space of the casing 2.

Meanwhile, the plate-like portion of the load receiving unit 17 is integrally formed with the cylindrical structure of the load receiving unit 17 at one opening end thereof, and as shown in FIG. 1, includes the power supply board 18b and the contact spring 13b on the surface facing the battery 12b. The plate-like portion of the load receiving unit 17 has a through hole for preventing a contact with the circuit components such as the capacitor mounted on the control board 19c, arranged in the space formed by the cylindrical structure of the load receiving unit 17. The plate-like portion of the load receiving unit 17 receives the elastic force (that is, biasing force that acts in a direction of pressing the positioning unit 15 to the inner circumference of the optical dome 2c) of the contact spring 13b generated with contraction of the contact spring 13b, and presses the cylindrical structure of the load receiving unit 17 to the opening end of the protrusion 15b of the positioning unit 15 by the elastic force of the contact spring 13b.

The load receiving unit 17 having the cylindrical structure and the plate-like portion presses and fixes the flange of the protrusion 15b to the opening end of the optical dome 2c by the elastic force of the contact spring 13b. In this case, the load receiving unit 17 presses and fixes the protrusion 15b to the opening end of the optical dome 2c, thereby fitting and fixing the plate-like portion 15a integral with the protrusion 15b to a predetermined position on the inner circumference of the optical dome 2c.

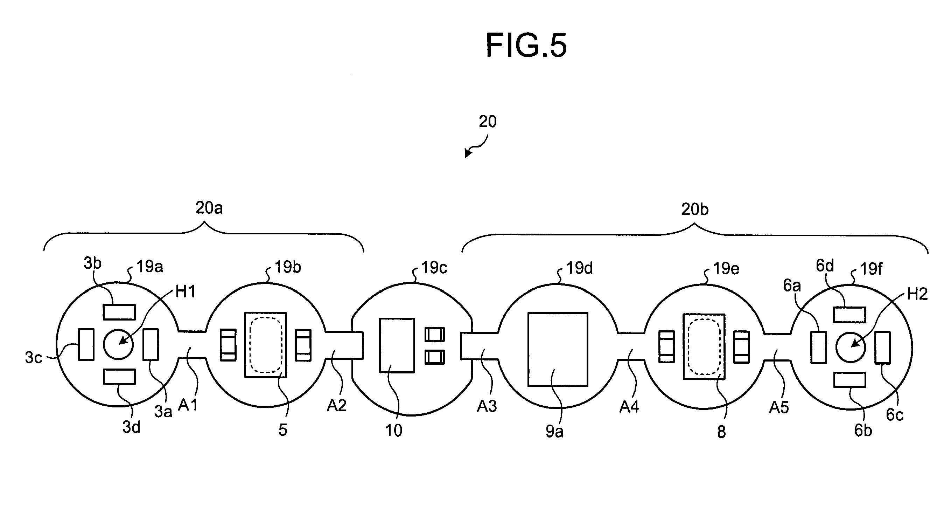

A series of circuit boards (specifically, the illuminating boards 19a and 19f, the imaging boards 19b and 19e, the control board 19c, and the wireless board 19d) arranged in the casing 2 of the capsule endoscope 1 is explained next. FIG. 5 is a schematic diagram for exemplifying a state where the series of circuit boards folded and arranged in the casing 2 of the capsule endoscope 1 is developed. Each board surface of the flexible board or the rigid board shown in FIG. 5 is defined as a board surface at the front (front board surface), and a back face of the front board surface shown in FIG. 5 is defined as a board surface at the back (back board surface).

As shown in FIG. 5, a series of circuit boards 20 arranged in the casing 2 of the capsule endoscope 1 is achieved by electrically connecting a series of flexible boards 20a connecting the illuminating board 19a and the imaging board 19b, the control board 19c as the rigid board, and a series of flexible boards 20b connecting the wireless board 19d, the imaging board 19e, and the illuminating board 19f.

The illuminating board 19a is flexible board having a substantially disk shape, on which a circuit for realizing an illuminating function for illuminating the subject on the direction F side of the capsule endoscope 1 is formed. The plurality of light-emitting elements 3a to 3d are mounted on the front board surface of the illuminating board 19a, and an opening part H1 for inserting the lens frame 4d of the optical unit 4 having the lens 4b, in a manner in which the legs thereof abut against the solid-state imaging device 5, is formed at the center of the board surface of the illuminating board 19a surrounded by the light-emitting elements 3a to 3d. The illuminating board 19a is electrically connected to the imaging board 19b via an extending part A1, which is a flexible board extending from an outer edge.

The imaging board 19b is a flexible board having a substantially disk shape, on which a circuit for realizing the imaging function for capturing the in-vivo image on the direction F side is formed. The solid-state imaging device 5 is flip-chip mounted on the front board surface of the imaging board 19b, and the circuit components such as the capacitor are mounted thereon as required. As shown by a dotted line in FIG. 5, in the imaging board 19b, there is formed an opening part for the reflected light from inside of the subject on the direction F side to enter into a light-receiving surface of the flip-chip mounted solid-state imaging device 5. Although not specifically shown in FIG. 5, the lower end of the lens frame 4d of the optical unit 4 abutting against the legs of the lens 4b is fixed on the light-receiving side device surface of the solid-state imaging device 5 via the opening part of the imaging board 19b, as shown in FIG. 1. The imaging board 19b is electrically connected to the control board 19c via an extending part A2, which is a flexible board extending from the outer edge.

The control board 19c is a rigid board having a substantially disk shape, on which a circuit necessary for the power supply system such as the magnetic switch 11a and the control unit 10 is formed. The control unit 10 is mounted on the front board surface of the control board 19c, and the circuit components such as the capacitor are mounted thereon as required. Meanwhile, as shown in FIG. 4, the magnetic switch 11a, the capacitors 11b and 11c, and the power supply IC 11d, which are the circuit components of the power supply system, are mounted on the back board surface of the control board 19c. The control board 19c is electrically connected to the wireless board 19d via an extending part A3, which is a flexible board extending from the outer edge of the wireless board 19d. Although not specifically shown in FIG. 5, the control board 19c is electrically connected to the power supply boards 18a and 18b via the flexible board or the like (not shown).

The wireless board 19d is a flexible board having a substantially disk shape, on which a circuit for realizing the wireless communication function for wirelessly transmitting the in-vivo image on the direction F side and the in-vivo image on the direction B side sequentially to the outside is formed. The wireless unit 9a is mounted on the front board surface of the wireless board 19d. Although not particularly shown in FIG. 5, the wireless board 19d is electrically connected to the antenna 9b fixed and arranged on the outer edge of the illuminating board 19f, as shown in FIGS. 1 and 3. The wireless board 19d is electrically connected to the imaging board 19e via an extending part A4, which is a flexible board extending from the outer edge.

The imaging board 19e is a flexible board having a substantially disk shape, on which a circuit for realizing the imaging function for capturing the in-vivo image on the direction B side is formed. The solid-state imaging device 8 is flip-chip mounted on the front board surface of the imaging board 19e, and the circuit components such as the capacitor are mounted as required. As shown by a dotted line in FIG. 5, in the imaging board 19e, there is formed an opening part for the reflected light from inside of the subject on the direction F side to enter into a light-receiving surface of the flip-chip mounted solid-state imaging device 8. Although not specifically shown in FIG. 5, the lower end of the lens frame 7d of the optical unit 7 abutting against the legs of the lens 7b is fixed on the light-receiving side device surface of the solid-state imaging device 8 via the opening part of the imaging board 19e, as shown in FIG. 1. The imaging board 19e is electrically connected to the illuminating board 19f via an extending part A5, which is a flexible board extending from the outer edge.

The illuminating board 19f is a flexible board having a substantially disk shape, on which a circuit that realizes the illuminating function for illuminating the subject on the direction B side of the capsule endoscope 1 is formed. The light-emitting elements 6a to 6d described above are mounted on the front board surface of the illuminating board 19f, and an opening part H2 for inserting the lens frame 7d of the optical unit 7 having the lens 7b in a manner in which the legs abut against the solid-state imaging device 8 is formed at the center of the board surface of the illuminating board 19f surrounded by the light-emitting elements 6a to 6d.

The series of flexible boards 20a is an integrally formed flexible board including the illuminating board 19a and the imaging board 19b, and has a board structure in which the imaging board 19b having the extending part A2 for connecting to the control board 19c extending from the outer edge thereof is connected to the illuminating board 19a via the extending part A1. On the other hand, the series of flexible boards 20b is an integrally formed flexible board including the wireless board 19d, the imaging board 19e, and the illuminating board 19f, and has a board structure in which the wireless board 19d having the extending part A3 for connecting to the control board 19c extending from the outer edge thereof is connected to the imaging board 19e via the extending part A4, and a board structure in which the imaging board 19e and the illuminating board 19f are connected to each other via the extending part A5. The series of circuit boards 20 arranged in the casing 2 of the capsule endoscope 1 is realized by connecting the series of flexible boards 20a and 20b with the control board 19c via the extending parts A2 and A3.

A manufacturing method of the capsule endoscope 1 according to the embodiment of the present invention is explained next. The capsule endoscope 1 is manufactured by preparing the series of circuit boards 20 shown in FIG. 5, preparing a functional unit by combining the manufactured series of circuit boards 20, the positioning units 14 and 15, the load receiving units 16 and 17, and the batteries 12a and 12b, and arranging the manufactured functional unit in the casing 2.

Specifically, the necessary functional components are first mounted on the illuminating board 19a and the imaging board 19b in the series of flexible boards 20a, and the necessary functional components are then mounted on the wireless board 19d, the imaging board 19e, and the illuminating board 19f in the series of flexible boards 20b. In this case, in the series of flexible boards 20a, the plurality of light-emitting elements 3a to 3d are mounted on the front board surface of the illuminating board 19a, the solid-state imaging device 5 and the circuit components such as the capacitor are mounted on the front board surface of the imaging board 19b, and the optical unit 4 is mounted on the back board surface of the imaging board 19b in a manner in which the legs of the lens 4b abut against the solid-state imaging device 5. Further, in the series of flexible boards 20b, the plurality of light-emitting elements 6a to 6d and the antenna 9b are mounted on the front board surface of the illuminating board 19f, the solid-state imaging device 8 and the circuit components such as the capacitor are mounted on the front board surface of the imaging board 19e, and the optical unit 7 is mounted on the back board surface of the imaging board 19e in a manner in which the legs of the lens 7b abut against the solid-state imaging device 8. Meanwhile, the control unit 10 and the circuit components such as the capacitor are mounted on the front board surface of the control board 19c, and the circuit components of the power supply system such as the magnetic switch 11a are mounted on the back board surface of the control board 19c. The series of circuit boards 20 is manufactured by connecting the series of flexible boards 20a and 20b.

The lens frame 4d of the optical unit 4 is a separate body with respect to the positioning unit 14, and is mounted on the back board surface of the imaging board 19b before being fitted and fixed in a through hole of the positioning unit 14 (specifically, the plate-like portion 14a) as shown in FIG. 1. Therefore, a working space required for applying an adhesive to a clearance between the imaging board 19b and the lower end of the lens frame 4d can be ensured sufficiently, and the lens frame 4d can be easily fixed to the imaging board 19b by the adhesive. The same applies to the lens frame 7d fitted to the back board surface of the imaging board 19e.

The functional unit of the capsule endoscope 1 is then manufactured by combining the series of circuit boards 20 manufactured as described above, the positioning units 14 and 15, the load receiving units 16 and 17, and the batteries 12a and 12b. The functional unit is the one excluding the casing 2 of the capsule endoscope 1 shown in FIG. 1 (that is, a built-in unit arranged in the casing 2). An external diameter of the functional unit, which is the built-in unit, is smaller than the internal diameter of the cylindrical body 2a of the casing 2, and thus backward and forward movement (movement to the direction F and direction B shown in Fig. 1) of the functional unit is not blocked by the inner circumference of the cylindrical body 2a. The external diameter of the functional unit is defined by the external diameter of the flanges of the positioning units 14 and 15, and the external diameter of the cylindrical structure of the load receiving unit 17.

In the functional unit, the lens frame 4d of the optical unit 4 mounted on the imaging board 19b is fitted and fixed in a through hole formed in the plate-like portion 14a of the positioning unit 14. An adhesive or a double-sided tape is applied or attached to one surface of the plate-like portion 14a (a surface facing the optical dome 2b) as a bonding member, and the illuminating board 19a is fixed to the plate-like portion 14a by the bonding member, with the lens frame 4d being inserted into the opening part H1. The outer edge of the load receiving unit 16 is engaged with the protrusion 14b of the positioning unit 14, to which the illuminating board 19a and the imaging board 19b are fitted. In this case, the load receiving unit 16 is fitted to the protrusion 14b in a manner in which the power supply board 18a and the contact spring 13a are arranged on the backward side of the surface facing the solid-state imaging device 5 of the imaging board 19b.

Meanwhile, the lens frame 7d of the optical unit 7 mounted on the imaging board 19e is fitted and fixed in the through hole formed in the plate-like portion 15a of the positioning unit 15. The adhesive or double-sided tape is applied or attached to one surface of the plate-like portion 15a (a surface facing the optical dome 2c) as a bonding member, and the illuminating board 19f is fixed to the plate-like portion 15a by the bonding member, with the lens frame 7d being inserted into the opening part H2. An end of the cylindrical structure of the load receiving unit 17 is engaged with the protrusion 15b of the positioning unit 15, to which the illuminating board 19f and the imaging board 19e are fitted. In this case, the load receiving unit 17 is fitted to the protrusion 15b in a state where the control board 19c and the wireless board 19d are arranged in the space formed by the cylindrical structure, and the power supply board 18b and the contact spring 13b can be arranged to face the power supply board 18a and the contact spring 13a of the load receiving unit 16.

The load receiving unit 17 ensures the space for arranging the control board 19c and the wireless board 19d in the casing 2, and supports the control board 19c and the wireless board 19d. Therefore, board spacing between the control board 19c and the wireless board 19d can be ensured by the load receiving unit 17, without filling a filler such as an adhesive in the casing as in the conventional capsule endoscope. The load receiving unit 17 can hold an assembly shape of the functional unit of the capsule endoscope 1 so as not to deviate in a radial direction of the casing 2, by bringing and fitting the end thereof into contact with and to the flange. Therefore, in the method of manufacturing the capsule endoscope 1 according to the first embodiment of the present invention, a process of filling the filler in the casing 2 to ensure the space between the circuit boards can be omitted, thereby simplifying a process of manufacturing the capsule endoscope 1 and facilitating weight saving of the capsule endoscope 1.

Meanwhile, the batteries 12a and 12b are arranged between the load receiving units 16 and 17, in which the power supply board 18b and the contact spring 13b face the power supply board 18a and the contact spring 13a. In this case, the batteries 12a and 12b are held by the protrusion 14b of the positioning unit 14 an the end of the load receiving unit 17, with a positive pole and a negative pole thereof coming in contact with each other. The batteries 12a and 12b cause the contact springs 13a and 13b to contract, and are electrically connected to the power supply boards 18a and 18b via the contact springs 13a and 13b.

The functional unit of the capsule endoscope 1 is manufactured as described above. The series of circuit boards 20 incorporated in the functional unit is folded in a predetermined manner. In this case, as shown in FIG. 1, the back board surface of the illuminating board 19a and the back board surface of the imaging board 19b face each other via the plate-like portion 14a of the positioning unit 14, and the front board surface of the imaging board 19b and the front board surface of the control board 19c face each other via the load receiving units 16 and 17 and the batteries 12a and 12b. Further, the back board surface of the control board 19c and the back board surface of the wireless board 19d face each other, the front board surface of the wireless board 19d and the front board surface of the imaging board 19e face each other, and the back board surface of the imaging board 19e and the back board surface of the illuminating board 19f face each other via the plate-like portion 15a of the positioning unit 15. The extending part A1 is inserted into a notch (not shown) formed in the positioning unit 14, and the extending part A2 is inserted into notches (not shown) formed in the protrusion 14b of the positioning unit 14 and the load receiving unit 17. The extending part A3 is inserted into a notch (not shown) formed in the cylindrical structure of the load receiving unit 17, the extending part A4 is inserted into notches (not shown) formed in the opening end of the load receiving unit 17 and the protrusion 15b of the positioning unit 14, and the extending part A5 is inserted into a notch (not shown) formed in the positioning unit 15.

Thereafter, the functional unit described above is arranged in the capsule casing 2. That is, the functional unit is inserted into the cylindrical body 2a, and the optical domes 2b and 2c are fitted to respective inner circumferences near the both opening ends of the cylindrical body 2a, which houses the functional unit. In this case, as shown in Fig. 1, the optical domes 2b and 2c are fitted to the respective inner circumferences near the both opening ends of the cylindrical body 2a and fixed by engaging between the protrusions on the inner circumference of the cylindrical body 2a and the depressions on the respective outer circumferences of the optical domes 2b and 2c and the adhesive or the like. Accordingly, the capsule endoscope 1 as shown in Fig. 1 is completed.

A liquid-tight state of the casing 2 of the capsule endoscope 1 is realized by filling the adhesive in the space between the inner circumference of the cylindrical body 2a and the outer circumferences of the optical domes 2b and 2c. A retaining force between the cylindrical body 2a and the optical domes 2b and 2c by engaging between the protrusions of the cylindrical body 2a and the depressions of the optical domes 2b and 2c is a force repulsive to the elastic force of the contact springs 13a and 13b for pressing the positioning units 14 and 15 to the optical domes 2b and 2c, and is larger than the elastic force of the contact springs 13a and 13b. Therefore, the optical domes 2b and 2c do not detach from the cylindrical body 2a due to the elastic force of the contact springs 13a and 13b. The adhesive filled in the space between the cylindrical body 2a and the optical domes 2b and 2c is for supplementing (reinforcing) the retaining force between the cylindrical body 2a and the optical domes 2b and 2c, and reliably fixes the cylindrical body 2a and the optical domes 2b and 2c.

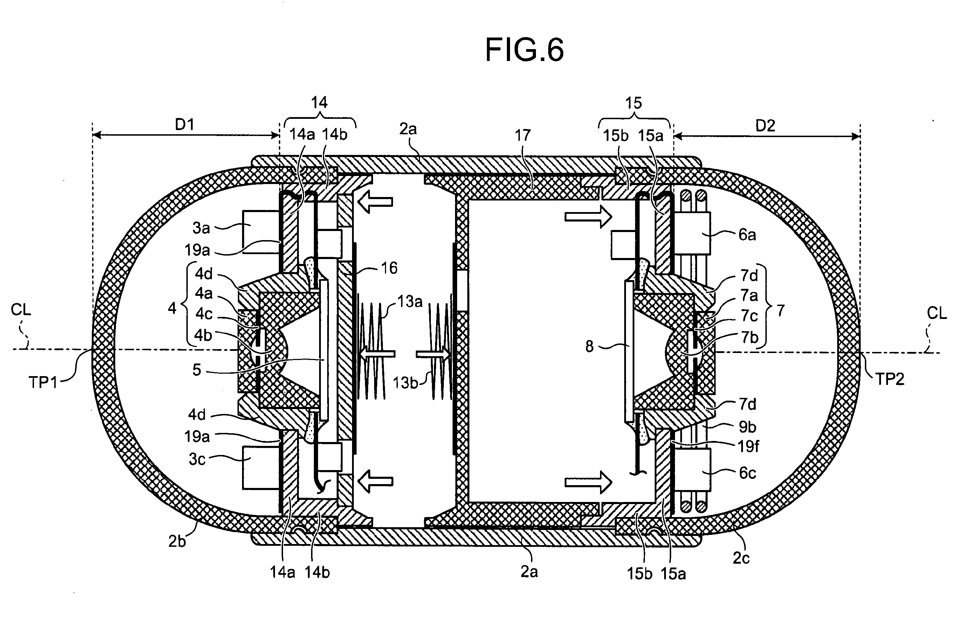

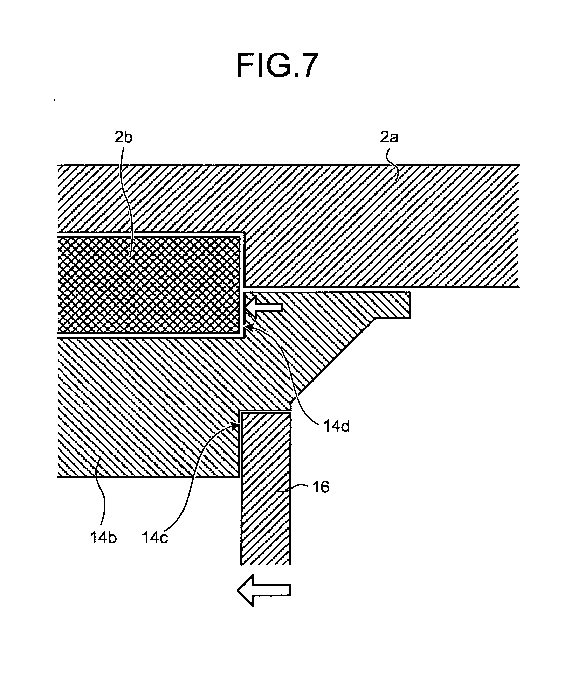

A positioning effect of the light-emitting elements 3a to 3d and the optical unit 4 by the positioning unit 14 and a positioning effect of the light-emitting elements 6a to 6d and the optical unit 7 by the positioning unit 15 are explained next. Fig. 6 is a schematic diagram for explaining the positioning effect of the light-emitting elements and the optical unit with respect to the optical domes. Fig. 7 is a schematic diagram for exemplifying an engaging state of the opening end of the optical dome 2b and the load receiving unit 16 with respect to the flange of the positioning unit 14.

As shown in Fig. 1, when the optical domes 2b and 2c are respectively fitted and fixed to the both opening ends of the cylindrical body 2a, the opening end of the optical dome 2b engages with the step formed on the inner circumference of the cylindrical body 2a and the step on the outer circumference (that is, the flange) of the protrusion 14b, and the opening end of the optical dome 2c engages with the step formed on the inner circumference of the cylindrical body 2a and the step on the outer circumference (that is, the flange) of the protrusion 15b. In this case, the contact spring 13a is compressed from the load receiving unit 16 side and the battery 12a side to contract, and reliably ensures a contact point between the battery 12a and the power supply board 18a and generates the elastic force. Similarly, the contact spring 13b is compressed from the load receiving unit 17 side and the battery 12a side to contract, thereby surely ensuring a contact point between the battery 12b and the power supply board 18b and generate the elastic force.

The contact spring 13a is an example of an elastic member that generates a biasing force for pressing the positioning unit 14 to the optical dome 2b, and the contact spring 13b is an example of the elastic member that generates the biasing force for pressing the positioning unit 15 to the optical dome 2c. The biasing force (that is, elastic force) of the contact springs 13a and 13b is a force acting in an opposite direction to each other toward the respective solid-state imaging devices 5 and 8, and is smaller than the retaining force between the cylindrical body 2a and the optical domes 2b and 2c. A distance for pushing the load receiving units 16 and 17 by the elastic force of the contact springs 13a and 13b (that is, an expansion and contraction distance of the contact springs 13a and 13b) is larger than a size variation of the functional unit of the capsule endoscope 1 in an acting direction of the biasing force of the contact springs 13a and 13b, (specifically, a size variation of, for example, the positioning units 14 and 15 and the load receiving units 16 and 17).

The contact spring 13a that has generated the elastic force applies the generated elastic force to the load receiving unit 16, as shown in Fig. 6. The load receiving unit 16 receives the elastic force of the contact spring 13a. As shown in Figs. 6 and 7, the outer edge of the load receiving unit 16 engages with the engaging unit 14c, which is a step formed on the inner circumference of the protrusion 14b of the positioning unit 14. The load receiving unit 16 presses the protrusion 14b in a direction of pressing the protrusion 14b to the opening end of the optical dome 2b (a direction of a thick arrow shown in Fig. 7) by the elastic force of the contact spring 13a.

An appropriate clearance is formed between the outer circumference of the positioning unit 14 and the inner circumference of the cylindrical body 2a, and an appropriate clearance is formed between the outer circumference of the positioning unit 14 and the inner circumference of the optical dome 2b. The appropriate clearance is as small as making the positioning unit 14 slidable with respect to the cylindrical body 2a or the optical dome 2b, but not so large as increasing the variation in the relative position between the optical dome 2b and the positioning unit 14 to generate flare at the time of capturing an image. Because the positioning unit 14 is slidable with respect to the respective inner circumferences of the cylindrical body 2a and the optical dome 2b, a loss of the elastic force of the contact spring 13a due to friction or the like between the cylindrical body 2a or the optical dome 2b and the positioning unit 14 can be reduced. A distance of the load receiving unit 16 pushed by the elastic force of the contact spring 13a is larger than the size variation of the functional unit of the capsule endoscope 1 in an acting direction of the elastic force of the contact spring 13a (for example, in a direction of a central axis CL). Accordingly, the elastic force of the contact spring 13a is reliably transmitted to the positioning unit 14 via the load receiving unit 16. Therefore, the load receiving unit 16 can easily and reliably press the protrusion 14b of the positioning unit 14 to the opening end of the optical dome 2b by the elastic force of the contact spring 13a. The protrusion 14b is fixed in a state with a flange 14d, which is a step formed on the outer circumference thereof, being pressed to the opening end of the optical dome 2b due to the operation of the load receiving unit 16.

The plate-like portion 14a integral with the protrusion 14b is fitted and fixed to a predetermined position on the inner circumference of the optical dome 2b due to the operation of the load receiving unit 16. That is, the positioning unit 14 fixes the relative position in the radial direction (in a direction perpendicular to the central axis CL) with respect to the optical dome 2b by fitting between the plate-like portion 14a having an external diameter designed to match with the internal diameter of the optical dome 2b and the optical dome 2b, and fixes the relative position in a direction of the central axis CL (in a longitudinal axis direction of the casing 2) with respect to the optical dome 2b by the elastic force of the contact spring 13a (a pressing force of the load receiving unit 16) transmitted via the load receiving unit 16 described above. The positioning unit 14 determines the respective relative positions of the light-emitting elements 3a to 3d, the optical unit 4 or the like with respect to the optical dome 2b.

Specifically, as shown in Fig. 6, the positioning unit 14 fixes the relative position in the radial direction with respect to the optical dome 2b so that the optical axis of the optical unit 4 approximately matches the central axis CL, and fixes the relative position in the direction of the central axis CL with respect to the optical dome 2b so that the illuminating board 19a is fixedly arranged at a position of distance D1 from a top part TP1 of the optical dome 2b. The positioning unit 14 fixes the positional relation between the light-emitting elements 3a to 3d on the illuminating board 19a, the optical unit 4, and the optical dome 2b, and determines preferable relative positions of the light-emitting elements 3a to 3d and the optical unit 4 with respect to the optical dome 2b highly accurately.

Because the external structure of the positioning unit 14 is formed by injection molding of a resin, different from the rigid board formed by punching or the like, an external diameter tolerance can be reduced (for example, to ± 0.5 millimeter or less) as compared with that of the rigid board (for example, ± 0.1 millimeter). The external structure of the positioning unit 14 capable of being fitted and fixed to the optical dome 2b by the elastic force of the contact spring 13a is, for example, an outer circumferential structure of the plate-like portion 14a having the external diameter designed to match with the internal diameter of the optical dome 2b or a flange structure of the protrusion 14b capable of engaging with the opening end of the optical dome 2b. Accordingly, the positioning unit 14 can control the variation in the relative positional relation between the optical dome 2b, the light-emitting elements 3a to 3d, and the optical unit 4, and can fixedly arrange the respective light-emitting elements 3a to 3d and the optical unit 4 at respective preferable relative positions with respect to the optical dome 2b highly accurately by being fitted and fixed to the optical dome 2b. As a result, flare generated due to the light from the light-emitting elements 3a to 3d reflected by the optical dome 2b and entering into the lenses 4a and 4b of the optical unit 4 can be prevented.

The positioning unit 14 can determine the respective preferable relative positions of the solid-state imaging device 5 and the imaging board 19b with respect to the optical dome 2b highly accurately, as in the light-emitting elements 3a to 3d and the optical unit 4. As a result, the space required for arranging the solid-state imaging device 5 and the imaging board 19b can be suppressed to the minimum, thereby enabling to facilitate downsizing of the casing 2.

On the other hand, the contact spring 13b that has generated the elastic force applies the generated elastic force to the load receiving unit 17, as shown in Fig. 6. The load receiving unit 17 receives the elastic force of the contact spring 13b. As shown in Fig. 6, the cylindrical structure of the load receiving unit 17 engages with the opening end of the protrusion 15b of the positioning unit 15. The load receiving unit 17 presses the flange of the protrusion 15b to the opening end of the optical dome 2c by the elastic force of the contact spring 13b.

An appropriate clearance is formed between the outer circumference of the positioning unit 15 and the inner circumference of the cylindrical body 2a, and an appropriate clearance is formed between the outer circumference of the positioning unit 15 and the inner circumference of the optical dome 2c. The appropriate clearance is as small as making the positioning unit 15 slidable with respect to the cylindrical body 2a or the optical dome 2c, but not so large as increasing the variation in the relative position between the optical dome 2c and the positioning unit 15 to generate flare at the time of capturing an image. Because the positioning unit 15 is slidable with respect to the respective inner circumferences of the cylindrical body 2a and the optical dome 2c, a loss of the elastic force of the contact spring 13b due to friction or the like between the cylindrical body 2a or the optical dome 2c and the positioning unit 15 can be reduced. A distance of the load receiving unit 17 pushed by the elastic force of the contact spring 13b is larger than the size variation of the functional unit of the capsule endoscope 1 in the acting direction of the elastic force of the contact spring 13b (for example, in the direction of the central axis CL). Accordingly, the elastic force of the contact spring 13b is reliably transmitted to the positioning unit 15 via the load receiving unit 17. Therefore, the load receiving unit 17 can easily and reliably press the protrusion 15b of the positioning unit 15 to the opening end of the optical dome 2c by the elastic force of the contact spring 13b. The protrusion 15b is fixed in a state with the flange, which is a step formed on the outer circumference thereof, being pressed to the opening end of the optical dome 2c due to the operation of the load receiving unit 17.

The plate-like portion 15a integral with the protrusion 15b is fitted and fixed to a predetermined position on the inner circumference of the optical dome 2c due to the operation of the load receiving unit 17. That is, the positioning unit 15 fixes the relative position in the radial direction with respect to the optical dome 2c by fitting between the plate-like portion 15a having an external diameter designed to match with the internal diameter of the optical dome 2c and the optical dome 2c, and fixes the relative position in the direction of the central axis CL with respect to the optical dome 2c by the elastic force of the contact spring 13b (a pressing force of the load receiving unit 17) transmitted via the load receiving unit 17. The positioning unit 15 determines the respective relative positions of the light-emitting elements 6a to 6d and the optical unit 7 with respect to the optical dome 2c.

Specifically, as shown in Fig. 6, the positioning unit 15 fixes the relative position in the radial direction with respect to the optical dome 2c so that the optical axis of the optical unit 7 approximately matches the central axis CL, and fixes the relative position in the direction of the central axis CL with respect to the optical dome 2c so that the illuminating board 19f is fixedly arranged at a position of distance D2 from a top part TP2 of the optical dome 2c. The positioning unit 15 fixes the positional relation between the light-emitting elements 6a to 6d on the illuminating board 19f, the optical unit 7, and the optical dome 2c, and determines preferable relative positions of the light-emitting elements 6a to 6d and the optical unit 7 with respect to the optical dome 2c highly accurately.

Because the external structure of the positioning unit 15 is formed by injection molding of the resin, as in the positioning unit 14, an external diameter tolerance can be reduced (for example, to ± 0.5 millimeter or less) as compared with that of the rigid board (for example, ± 0.1 millimeter). The external structure of the positioning unit 15 capable of being fitted and fixed to the optical dome 2c by the elastic force of the contact spring 13b is, for example, an outer circumferential structure of the plate-like portion 15a having the external diameter designed to match with the internal diameter of the optical dome 2c or a flange structure of the protrusion 15b capable of engaging with the opening end of the optical dome 2c. Accordingly, the positioning unit 15 can control the variation in the relative positional relation between the optical dome 2c, the light-emitting elements 6a to 6d, and the optical unit 7, and can fixedly arrange the respective light-emitting elements 6a to 6d and the optical unit 7 at respective preferable relative positions with respect to the optical dome 2c highly accurately by being fitted and fixed to the optical dome 2c. As a result, flare generated due to the light from the light-emitting elements 6a to 6d reflected by the optical dome 2c and entering into the lenses 7a and 7b of the optical unit 7 can be prevented.

The positioning unit 15 can determine the respective preferable relative positions of the solid-state imaging device 8 and the imaging board 19e with respect to the optical dome 2c highly accurately, as in the light-emitting elements 6a to 6d and the optical unit 7. As a result, the space required for arranging the solid-state imaging device 8 and the imaging board 19e can be suppressed to the minimum, thereby enabling to facilitate downsizing of the casing 2. Further, the positioning unit 15 can determine the preferable relative position of the antenna 9b with respect to the optical dome 2c highly accurately. As a result, the space required for arranging the antenna 9b on the illuminating board 19f can be suppressed to the minimum, thereby enabling to facilitate downsizing of the casing 2.

As explained above, in the capsule medical device according to the first embodiment of the present invention, the illuminating board having the light-emitting elements that illuminate inside of the subject over the optical dome and the optical unit that forms an image of inside the subject on the light receiving surface of the solid-state imaging device are fixedly arranged with respect to the positioning unit having the external structure capable of being fitted and fixed to the inner circumference of the optical dome. Further, the outer circumference of the positioning unit including the illuminating board and the optical unit fixedly arranged thereon is fitted and fixed to a predetermined position on the inner circumference of the optical dome (for example, by the biasing force of the elastic member) to determine the respective relative positions of the light-emitting element on the illuminating board and the optical unit with respect to the optical dome. Accordingly, variations in the relative positional relation of the light-emitting elements on the illuminating board and the optical unit with respect to the optical dome can be suppressed. As a result, the respective preferable relative positions of the light-emitting elements on the illuminating board (that is, the illuminating unit that illuminates the inside of the subject) and the optical unit with respect to the optical dome can be determined highly accurately.

The illuminating unit and the optical unit can be fixedly arranged highly accurately at the preferable relative positions with respect to the optical dome by using the capsule medical device according to the first embodiment of the present invention, thereby enabling to prevent the flare generated due to the light emitted from the illuminating unit, reflected by the optical dome, and entering into the lens of the optical unit.

In the capsule medical device according to the first embodiment of the present invention, because the flexible board is employed as the circuit boards such as the illuminating board, the imaging board, and the wireless board, downsizing and weight saving of the capsule medical device can be facilitated and the board cost can be reduced, as compared with the conventional capsule medical device using the rigid board as the circuit board.

Further, in the capsule medical device according to the first embodiment of the present invention, the respective relative positions of the imaging unit and the imaging board with respect to the optical dome are fixed by the positioning unit as in the illuminating unit and the optical unit. Accordingly, respective preferable relative positions of the imaging unit and the imaging board with respect to the optical dome can be determined highly accurately. As a result, the space in the casing required for arranging the imaging unit and the imaging board can be suppressed to the minimum, thereby enabling to facilitate downsizing of the casing.

In the capsule medical device according to the first embodiment of the present invention, the relative position between the optical dome and the antenna on the illuminating board is fixed by the positioning unit. The antenna is arranged on the outer edge of the illuminating board (for example, outside of the light-emitting elements), which is a dead space in the conventional capsule medical device. Accordingly, the preferable relative position of the antenna on the illuminating board with respect to the optical dome can be determined highly accurately, and the space, which has been the dead space in the conventional capsule medical device, can be efficiently used as the space for arranging the antenna. As a result, the antenna can be arranged in the space surrounded by the optical dome and the illuminating board, and the space in the optical dome required for arranging the antenna on the illuminating board can be suppressed to the minimum, thereby enabling to further facilitate downsize of the casing.

In the capsule medical device according to the first embodiment of the present invention, the spacer (the load receiving unit 17) is arranged in the casing to ensure the space for arranging the circuit boards (for example, the control board 19c and the wireless board 19d) by the spacer, and the spacer supports the circuit boards to ensure the space between the respective circuit boards. Therefore, the space between the respective circuit boards can be ensured by the spacer that supports the circuit boards without filling the filler such as the adhesive in the casing, as in the conventional capsule medical device. As a result, the process of filling the filler in the casing can be omitted, thereby simplifying the process of manufacturing the capsule medical device and facilitating weight saving of the capsule medical device.

Further, because the retaining force of the cylindrical body and the optical dome constituting the casing of the capsule medical device according to the first embodiment of the present invention exceeds the biasing force of the elastic member that presses the positioning unit to the optical dome, it can be prevented that the optical dome detaches or floats from the cylindrical body due to the biasing force of the elastic member.

(First Modification) A capsule medical device according to a first modification of the first embodiment of the present invention is explained next. In the capsule endoscope 1 according to the first modification, the positioning units 14 and 15 and the respective lens frames 4d and 7d of the optical units 4 and 7 are separate bodies; however, in the capsule endoscope according to the first modification of the first embodiment, the positioning unit and the lens frame of the optical unit are integrated.

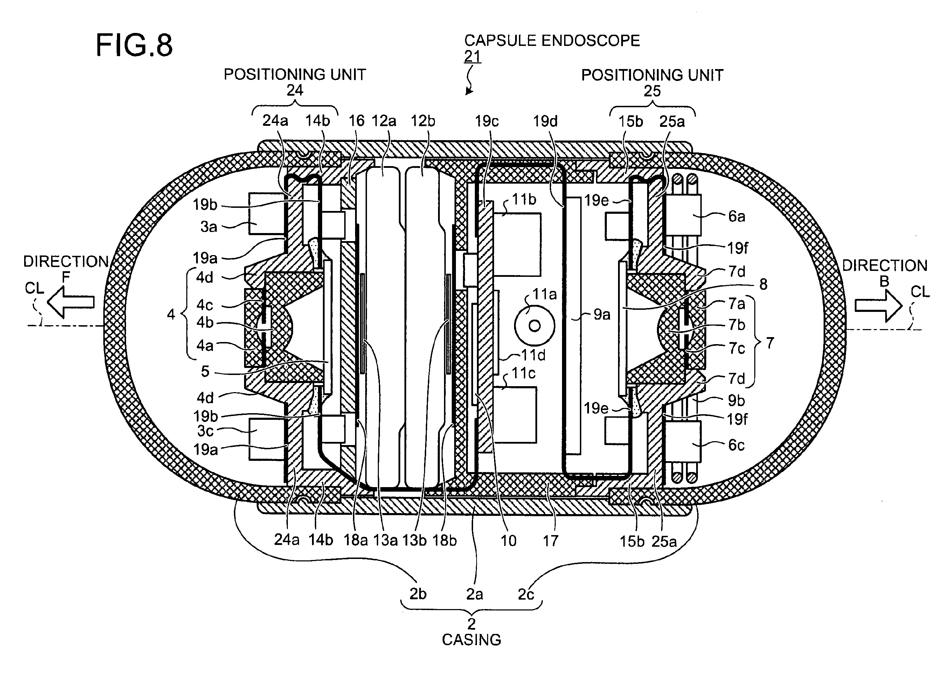

Fig. 8 is a schematic longitudinal cross section of a configuration example of a capsule endoscope according to the first modification of the first embodiment of the present invention. As shown in Fig. 8, a capsule endoscope 21 according to the first modification includes positioning units 24 and 25 integrally provided with a lens frame of the optical unit instead of the positioning units 14 and 15 of the capsule endoscope 1 according to the first embodiment. Other configurations of the first modification are the same as those of the first embodiment, and the same reference numerals are given to the same parts.

The positioning unit 24 includes a plate-like portion 24a obtained by integrating the plate-like portion 14a and the lens frame 4d of the optical unit 4 and the protrusion 14b, and has a positioning function similar to that of the positioning unit 14 of the capsule endoscope 1 according to the first embodiment. The plate-like portion 24a of the positioning unit 24 is the same as the plate-like portion 14a of the positioning unit 14, except for having the lens frame 4d of the optical unit 4 integrated therewith. That is, the plate-like portion 24a is formed by injection molding of a resin to have an outer circumferential structure having an external diameter designed to match with the internal diameter of the optical dome 2b (an external diameter for generating an appropriate clearance between the inner circumference of the optical dome 2b and the outer circumference of the plate-like portion 24a) and the lens frame 4d of the optical unit 4 integrated therewith. The plate-like portion 24a can be fitted and fixed to a predetermined position on the inner circumference of the optical dome 2b by the elastic force of the contact spring 13a as in the plate-like portion 14a. The positioning unit 24 including the plate-like portion 24a and the protrusion 14b can fixedly arrange the light-emitting elements 3a to 3d on the illuminating board 19a fixedly arranged on the plate-like portion 24a and lenses 4a and 4b held by the integrated lens frame 4d to respective preferable relative positions with respect to the optical dome 2b, as in the positioning unit 14.

The positioning unit 25 includes a plate-like portion 25a obtained by integrating the plate-like portion 15a and the lens frame 7d of the optical unit 7 and the protrusion 15b, and has a positioning function similar to that of the positioning unit 15 of the capsule endoscope 1 according to the first embodiment. The plate-like portion 25a of the positioning unit 25 is the same as the plate-like portion 15a of the positioning unit 15, except for having the lens frame 7d of the optical unit 7 integrated therewith. That is, the plate-like portion 25a is formed by injection molding of a resin to have an outer circumferential structure having an external diameter designed to match with the internal diameter of the optical dome 2c (an external diameter for generating an appropriate clearance between the inner circumference of the optical dome 2c and the outer circumference of the plate-like portion 25a) and the lens frame 7d of the optical unit 7 integrated therewith. The plate-like portion 25a can be fitted and fixed to a predetermined position on the inner circumference of the optical dome 2c by the elastic force of the contact spring 13b as in the plate-like portion 15a. The positioning unit 25 including the plate-like portion 25a and the protrusion 15b can fixedly arrange the light-emitting elements 6a to 6d on the illuminating board 19f fixedly arranged on the plate-like portion 25a and the lenses 7a and 7b held by the integrated lens frame 7d to respective preferable relative positions with respect to the optical dome 2c, as in the positioning unit 15.

As explained above, in the capsule medical device according to the first modification of the first embodiment of the present invention, the plate-like portion of the positioning unit and the lens frame of the optical unit are integrally formed, and other configurations of the first modification are the same as those of the first embodiment. Accordingly, the same operational effect as those of the first embodiment can be achieved, and the capsule medical device capable of reducing the number of components constituting the functional unit in the casing can be achieved. As a result, the process of manufacturing the capsule medical device can be simplified, and manufacturing cost can be reduced.

### (Second Modification)

A capsule medical device according to a second modification of the first embodiment of the present invention is explained next. In the capsule endoscope 1 according to the first embodiment, the positioning unit is fitted and fixed to the inner circumference of the optical dome by engaging the flange formed on the protrusion of the positioning unit with the opening end of the optical dome. However, in the capsule endoscope according to the second modification of the first embodiment, a plate-like positioning unit is fitted and fixed to the inner circumference of the optical dome, by engaging an outer edge of the positioning unit formed in a plate-like shape with a step formed on the inner circumference of the optical dome with each other.

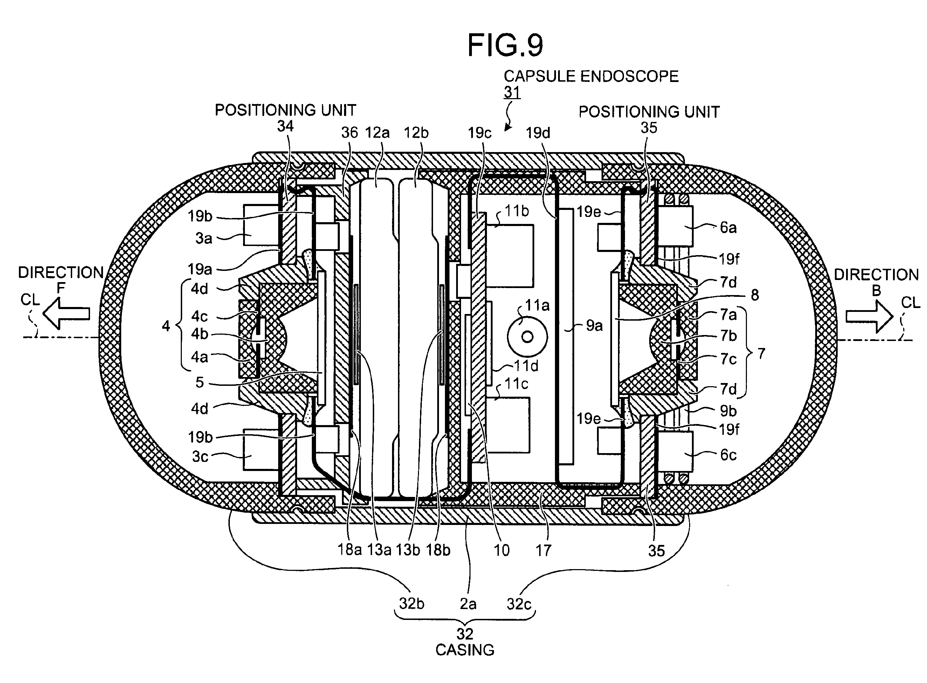

Fig. 9 is a schematic longitudinal cross section of a configuration example of a capsule endoscope according to the second modification of the first embodiment of the present invention. As shown in Fig. 9, a capsule endoscope 31 according to the second modification includes a casing 32 instead of the casing 2, positioning units 34 and 35 instead of the positioning units 14 and 15, and a load receiving unit 36 instead of the load receiving unit 16, of the capsule endoscope 1 according to the first embodiment. Other configurations of the second modification are the same as those of the first embodiment, and the same reference numerals are given to the same parts.

The casing 32 is a capsule casing having the same external structure as the casing 2 (capsule shape, external diameter, and the like) of the capsule endoscope 1 according to the first embodiment, and is achieved by fitting optical domes 32b and 32c to both opening ends of the cylindrical body 2a, instead of the optical domes 2b and 2c. The optical domes 32b and 32c are the same dome members as the optical domes 2b and 2c, except for having a step for fitting and fixing the plate-like positioning units 34 and 35 on an inner circumference thereof, respectively.

Specifically, as shown in Fig. 9, the optical dome 32b includes a first inner circumference to be fitted to an outer circumference of the plate-like positioning unit 34 and a second inner circumference having an internal diameter smaller than that of the first inner circumference, and has a step for connecting the first inner circumference and the second inner circumference at a predetermined position on the inner circumference side. The optical dome 32b fits and fixes the positioning unit 34 to a predetermined position on the inner circumference by engaging the outer edge of the positioning unit 34 with the step. Similarly, the optical dome 32c includes a first inner circumference for fitting the outer circumference of a plate-like positioning unit 35 thereon and a second inner circumference having an internal diameter smaller than that of the first inner circumference, and has a step for connecting the first inner circumference and the second inner circumference at a predetermined position on the inner circumference side. The optical dome 32c fits and fixes the positioning unit 35 to a predetermined position on the inner circumference by engaging the outer edge of the positioning unit 35 with the step.

The positioning unit 34 is an approximately disk-like plate-like member having an external diameter (external diameter for generating an appropriate clearance between the inner circumference of the optical dome 32b and the outer circumference of the positioning unit 34) designed to match with an internal diameter formed by the first inner circumference of the optical dome 32b, and the illuminating board 19a and the optical unit 4 are fixedly arranged therein as in the positioning unit 14 of the capsule endoscope 1 according to the first embodiment. The positioning unit 34 has an outer circumference slidably fitted to the first inner circumference of the optical dome 32b, and determines respective preferable relative positions of the light-emitting elements 3a to 3d, the optical unit 4, the solid-state imaging device 5, and the imaging board 19b with respect to the optical dome 32b, as in the positioning unit 14, by fitting and fixing the outer circumference to a predetermined position on the inner circumference of the optical dome 32b. In this case, the outer edge of the plate-like positioning unit 34 is engaged to the step of the optical dome 32b by an operation of the load receiving unit 36 described later. The outer edge of the illuminating board 19a fixedly arranged on a surface of the positioning unit 34 can be put between the step of the optical dome 32b and the outer edge of the positioning unit 34. Accordingly, detachment or floating of the illuminating board 19a from the positioning unit 34 can be reliably prevented.

The positioning unit 35 is an approximately disk-like plate-like member having an external diameter (external diameter for generating an appropriate clearance between the inner circumference of the optical dome 32c and the outer circumference of the positioning unit 35) designed to match with an internal diameter formed by the first inner circumference of the optical dome 32c, and the illuminating board 19f and the optical unit 7 are fixedly arranged therein as in the positioning unit 15 of the capsule endoscope 1 according to the first embodiment. The positioning unit 35 has an outer circumference slidably fitted to the first inner circumference of the optical dome 32c, and determines respective preferable relative positions of the light-emitting elements 6a to 6d, the optical unit 7, the solid-state imaging device 8, the imaging board 19e, and the antenna 9b on the illuminating board 19f with respect to the optical dome 32c, as in the positioning unit 15, by fitting and fixing the outer circumference to a predetermined position on the inner circumference of the optical dome 32c. In this case, the outer edge of the plate-like positioning unit 35 is engaged to the step of the optical dome 32c by an operation of the load receiving unit 17 applied with the elastic force of the contact spring 13b. The outer edge of the illuminating board 19f fixedly arranged on a surface of the positioning unit 35 can be put between the step of the optical dome 32c and the outer edge of the positioning unit 35. Accordingly, detachment or floating of the illuminating board 19f from the positioning unit 35 can be reliably prevented.

The load receiving unit 36 has a plate-like portion having the power supply board 18a and the contact spring 13a fixedly arranged thereon on a surface thereof opposed to the battery 12a, and has a supporting unit that supports the battery 12a and a pressing unit that presses the positioning unit 34 on the outer edge of the plate-like portion. An external diameter of the load receiving unit 36 is designed to be smaller than the respective internal diameters of the cylindrical body 2a and the optical dome 32b, an appropriate clearance is formed between the outer circumference of the load receiving unit 36 and the inner circumference of the cylindrical body 2a, and an appropriate clearance is formed between the outer circumference of the load receiving unit 36 and the inner circumference of the optical dome 32b. The load receiving unit 36 holds the batteries 12a and 12b in cooperation with the load receiving unit 17, and upon reception of the elastic force of the contact spring 13a, slides in a direction approaching the positioning unit 34 (a direction F shown in Fig. 9) due to the elastic force, to press and fix the positioning unit 34 to the step of the optical dome 32b. A through hole for avoiding a contact with circuit components such as a capacitor on the imaging board 19b is formed, as in the load receiving unit 16 of the capsule endoscope 1 according to the first embodiment.

As shown in Fig. 9, the load receiving unit 17 of the capsule endoscope 31 according to the second modification presses the opening end thereof to the plate-like positioning unit 35, and presses and fixes the positioning unit 35 to the step of the optical dome 32c by sliding in a direction approaching the positioning unit 35 (a direction B shown in Fig. 9) by the elastic force of the contact spring 13b. Other configurations of the load receiving unit 17 are the same as those of the capsule endoscope 1 according to the first embodiment described above.

As described above, in the capsule medical device according to the second modification of the first embodiment of the present invention, the plate-like positioning unit, on which the illuminating board and the optical unit are fixedly arranged, is fitted to the inner circumference of the optical dome having the step that connects the first inner circumference capable of being fitted to the outer circumference of the plate-like positioning unit (that is, the plate-like portion) and the second inner circumference having the internal diameter smaller than that of the first inner circumference, and the step of the optical dome and the outer edge of the positioning unit are engaged with each other to fit and fix the positioning unit to a predetermined position on the inner circumference of the optical dome, and other configurations of the second modification are the same as those of the first embodiment. Accordingly, the same operational effect as those of the first embodiment can be achieved, and the capsule medical device in which the positioning unit can be easily designed can be realized.
As a result, reduction of the manufacturing cost of the capsule medical device can be facilitated.

Because the illuminating board can be put between the outer edge of the positioning unit and the step of the optical dome engaged with each other, detachment or floating of the illuminating board from the positioning unit can be reliably prevented. Accordingly, the illuminating board can be fixedly arranged reliably on the surface of the positioning unit opposed to the optical dome.

(Second Embodiment) A second embodiment of the present invention is explained next. In the capsule endoscope according to the second embodiment, an index for viewing whether the optical dome is floating from the cylindrical body is further formed at a predetermined position on the optical dome to be fitted to the cylindrical body.

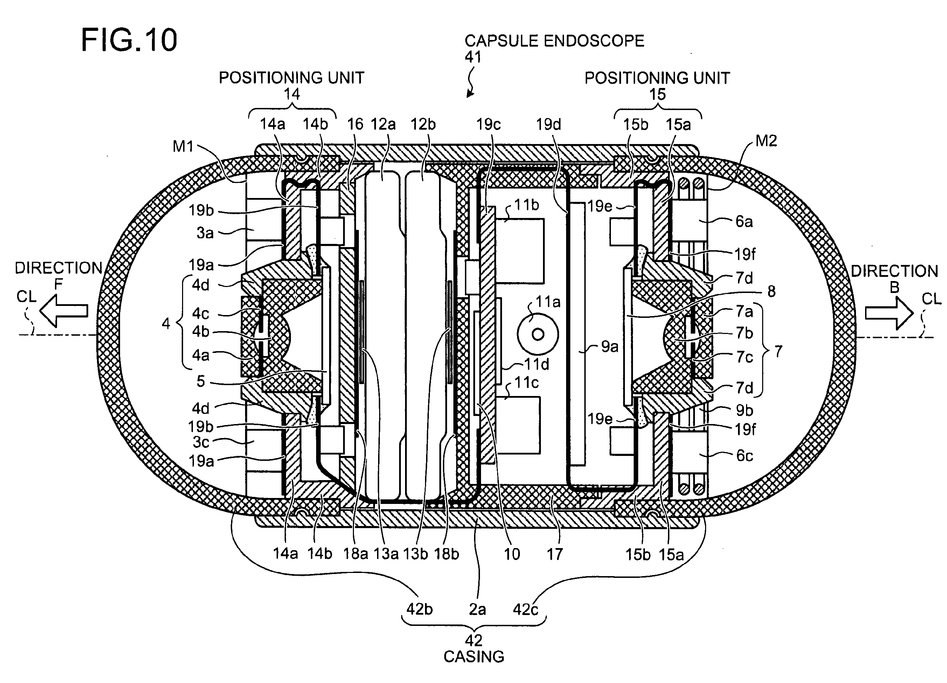

Fig. 10 is a schematic longitudinal cross section of a configuration example of a capsule endoscope according to the second embodiment of the present invention. As shown in Fig. 10, a capsule endoscope 41 according to the second embodiment includes a casing 42 instead of the casing 2 of the capsule endoscope 1 according to the first embodiment. The casing 42 includes optical domes 42b and 42c including indexes M1 and M2 formed thereon, instead of the optical domes 2b and 2c. Other configurations of the second embodiment are the same as those of the first embodiment, and the same reference numerals are given to the same parts.

The casing 42 is a capsule casing having the same external structure (capsule shape, external diameter, or the like) as that of the casing 2 of the capsule endoscope 1 according to the first embodiment, and is achieved by fitting the optical domes 42b and 42c instead of the optical domes 2b and 2c to the both opening ends of the cylindrical body 2a. The indexes M1 and M2 that can be viewable from outside are formed in the optical domes 42b and 42c, respectively. The optical domes 42b and 42c are the same dome members as the optical domes 2b and 2c, except that the indexes M1 and M2 are formed.

The indexes M1 and M2 are for viewing whether the optical domes 42b and 42c are floating from the cylindrical body 2a. The index M1 can be viewable from outside (for example, colored streaky or in a predetermined color), and is formed over the entire circumference in a circumferential direction of the optical dome 42b. Specifically, the index M1 is formed on a part of the optical dome 42b (for example, on the inner circumference or the outer circumference of the optical dome 42b that can be viewable from outside) not covered by the cylindrical body 2a at the time of normally attaching (fitting) the optical dome 42b to the inner circumference of the cylindrical body 2a, and outside of the imaging field of view of the solid-state imaging device 5. When the optical dome 42b is normally fitted to the cylindrical body 2a, for example, as shown in Fig. 10, the index M1 is positioned at the same height as the upper end of the light-emitting element in the casing 42. The light-emitting element with the upper end matched with the position of the index M1 is at least one of the light-emitting elements 3a to 3d.

The index M2 can be viewable from outside (for example, colored streaky or in a predetermined color), and is formed over the entire circumference in a circumferential direction of the optical dome 42c. Specifically, the index M2 is formed on a part of the optical dome 42c (for example, on the inner circumference or the outer circumference of the optical dome 42c that can be viewable from outside) not covered by the cylindrical body 2a at the time of normally attaching (fitting) the optical dome 42c to the inner circumference of the cylindrical body 2a, and outside of the imaging field of view of the solid-state imaging device 8. When the optical dome 42c is normally fitted to the cylindrical body 2a, for example, as shown in Fig. 10, the index M2 is positioned at the same height as the upper end of the light-emitting element in the casing 42. The light-emitting element with the upper end matched with the position of the index M2 is at least one of the light-emitting elements 6a to 6d.

When the optical domes 42b and 42c are normally fitted to the cylindrical body 2a, a protrusion on the inner circumference of the cylindrical body 2a and a depression on the outer circumference of the optical domes 42b and 42c are engaged with each other, and a step of the cylindrical body 2a and the opening ends of the optical domes 42b and 42c are brought into contact with each other. That is, when the optical dome 42b is floating from the cylindrical body 2a, the depression on the outer circumference of the optical dome 42b comes off from the protrusion on the inner circumference of the cylindrical body 2a, and the opening end of the optical dome 42b is detached from the step of the cylindrical body 2a. Similarly, when the optical dome 42c is floating from the cylindrical body 2a, the depression on the outer circumference of the optical dome 42c comes off from the protrusion on the inner circumference of the cylindrical body 2a, and the opening end of the optical dome 42c is detached from the step of the cylindrical body 2a.

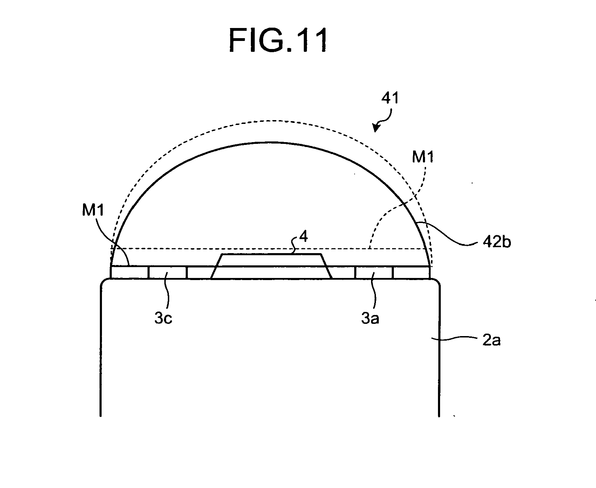

As shown in Fig. 11, when the index M1 is viewed from the side of the capsule endoscope 41 (that is, in a radial direction of the cylindrical body 2a), if the index M1 is substantially matched with the upper end of the light-emitting elements 3a and 3c, the optical dome 42b is not floating from the cylindrical body 2a and is normally fitted to the cylindrical body 2a. On the other hand, as shown by dotted line in Fig. 11, when the index M1 is not matched with the upper end of the light-emitting elements 3a and 3c, the optical dome 42b is floating from the cylindrical body 2a. Thus, it can be easily confirmed whether the optical dome 42b is floating from the cylindrical body 2a by viewing the position of the index M1 with respect to the built-in unit of the capsule endoscope 41 (for example, the upper ends of the light-emitting elements 3a and 3c).

Similarly, when the index M2 is viewed from the radial direction of the cylindrical body 2a, if the index M2 is substantially matched with the upper end of the light-emitting elements 6a and 6c, the optical dome 42c is not floating from the cylindrical body 2a and is normally fitted to the cylindrical body 2a. On the other hand, when the index M2 is not matched with the upper end of the light-emitting elements 6a and 6c, the optical dome 42c is floating from the cylindrical body 2a. Thus, it can be easily confirmed whether the optical dome 42c is floating from the cylindrical body 2a by viewing the position of the index M2 with respect to the built-in unit of the capsule endoscope 41 (for example, the upper ends of the light-emitting elements 6a and 6c).

In the capsule medical device according to the second embodiment of the present invention, the index that can be viewable from outside is formed in the optical dome when the capsule casing is formed by combining the optical dome and the cylindrical body, and when the optical dome is normally fitted to the cylindrical body, the index is substantially matched with a predetermined position in the casing, and other configurations of the second embodiment are the same as those in the first embodiment. Accordingly, the same operational effect as those of the first embodiment can be achieved, and it can be easily confirmed whether the optical dome is floating from the cylindrical body by viewing the relative position of the index with respect to the predetermined position in the casing. As a result, floating and detachment of the optical dome from the cylindrical body can be prevented, and generation of flare due to misregistration of the optical dome can be prevented.

### (Third Embodiment)

A third embodiment of the present invention is explained next. In a capsule endoscope according to the third embodiment, an O-ring is further arranged on the outer circumference of the optical dome fitted to the inner circumference of the cylindrical body, thereby ensuring a liquid-tight state of the casing by the O-ring.

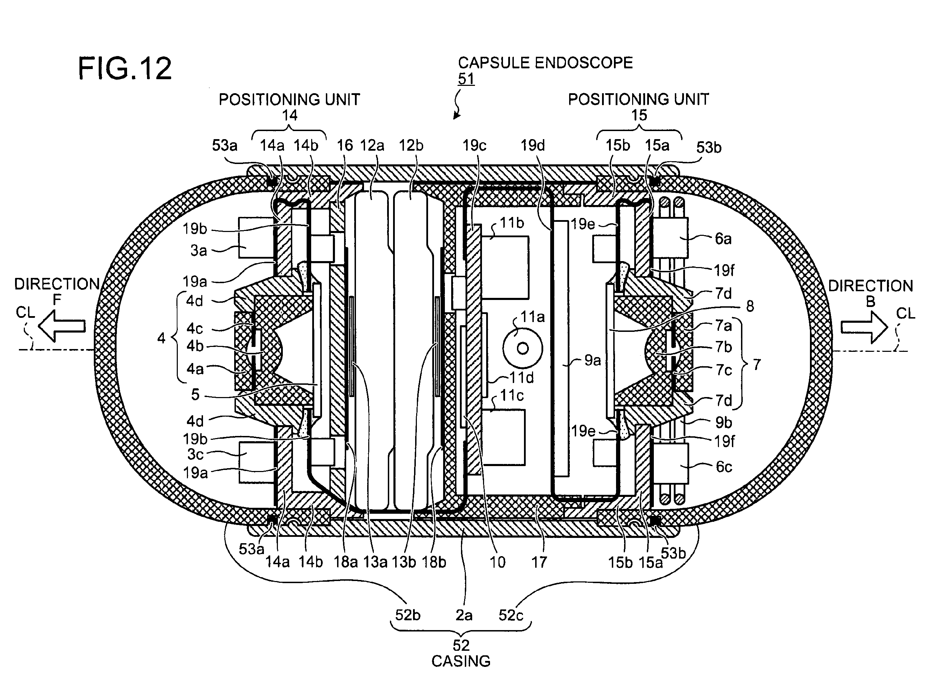

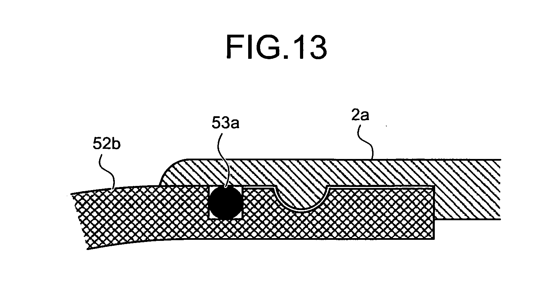

Fig. 12 is a schematic longitudinal cross section of a configuration example of the capsule endoscope according to the third embodiment of the present invention. Fig. 13 is a schematic longitudinal cross section for exemplifying a part of the optical dome where the O-ring is arranged. As shown in Figs. 12 and 13, a capsule endoscope 51 according to the third embodiment includes a casing 52 instead of the casing 2 of the capsule endoscope 1 according to the first embodiment. The casing 52 includes optical domes 52b and 52c, on which O-rings 53a and 53b are arranged on the outer circumference thereof, instead of the optical domes 2b and 2c. Other configurations of the third embodiment are the same as those of the first embodiment, and the same reference numerals are given to the same parts.

The casing 52 is a capsule casing having the same external structure (capsule shape, external diameter, or the like) as that of the casing 2 of the capsule endoscope 1 according to the first embodiment, and is realized by fitting the optical domes 52b and 52c instead of the optical domes 2b and 2c to the both opening ends of the cylindrical body 2a. The O-rings 53a and 53b are respectively arranged near the depressions on the optical domes 52b and 52c, which engage with the protrusions on the inner circumference of the cylindrical body 2a, on the outer circumference of the optical domes 52b and 52c. The optical domes 52b and 52c are fitted to the cylindrical body 2a, with the O-rings 53a and 53b being between the outer circumference of the optical domes and the inner circumference of the cylindrical body 2a. The optical domes 52b and 52c are the same dome members as the optical domes 2b and 2c, except for the O-rings 53a and 53b.

The O-rings 53a and 53b are put between the inner circumference of the cylindrical body 2a and the outer circumference of the optical domes 52b and 52c to ensure the liquid-tight state of the casing 52. The O-ring 53a is arranged on the outer circumference of the optical dome 52b covered by the inner circumference of the cylindrical body 2a at the time of fitting the optical dome 52b to the cylindrical body 2a. The O-ring 53a comes into contact with the inner circumference of the cylindrical body 2a over the entire circumference in the circumferential direction of the cylindrical body 2a to close the space between the inner circumference of the cylindrical body 2a and the outer circumference of the optical dome 52b, at the time of fitting the optical dome 52b to the cylindrical body 2a by engaging the protrusion on the inner circumference of the cylindrical body 2a with the depression on the outer circumference of the optical dome 52b. As a result, the O-ring 53a supplements the retaining force of the cylindrical body 2a and the optical dome 52b, and prevents a liquid from entering into the casing 52 via the space between the inner circumference of the cylindrical body 2a and the outer circumference of the optical dome 52b.

The O-ring 53b is arranged on the outer circumference of the optical dome 52c covered by the inner circumference of the cylindrical body 2a at the time of fitting the optical dome 52c to the cylindrical body 2a. The O-ring 53b comes into contact with the inner circumference of the cylindrical body 2a over the entire circumference in the circumferential direction of the cylindrical body 2a to close the space between the inner circumference of the cylindrical body 2a and the outer circumference of the optical dome 52c, at the time of fitting the optical dome 52c to the cylindrical body 2a by engaging the protrusion on the inner circumference of the cylindrical body 2a with the depression on the outer circumference of the optical dome 52c. As a result, the O-ring 53b supplements the retaining force of the cylindrical body 2a and the optical dome 52c, and prevents the liquid from entering into the casing 52 via the space between the inner circumference of the cylindrical body 2a and the outer circumference of the optical dome 52c.

The liquid-tight state of the casing 52 including the cylindrical body 2a and the optical domes 52b and 52c is ensured by using the O-rings 53a and 53b to close the space between the inner circumference of the cylindrical body 2a and the outer circumference of the optical dome 52b and the space between the inner circumference of the cylindrical body 2a and the outer circumference of the optical dome 52c, respectively.

In the conventional capsule endoscope in which the O-ring is not arranged in the optical dome, at the time of forming the capsule casing by fitting the optical dome to the cylindrical body, the adhesive is filled between the inner circumference of the cylindrical body and the outer circumference of the optical dome, to thereby fix the cylindrical body and the optical dome, and ensure the liquid-tight state of the casing by blocking the space between the inner circumference of the cylindrical body and the outer circumference of the optical dome. However, the work for filling (applying) the adhesive to between the inner circumference of the cylindrical body and the outer circumference of the optical dome requires a lot of skill, and there is a difference in liquid-tight performance of the casing due to subtle changes in an application amount of the adhesive. That is, it is difficult to ensure the liquid-tight state of the casing, if an appropriate amount of adhesive is not applied to between the inner circumference of the cylindrical body and the outer circumference of the optical dome.

On the other hand, in the capsule endoscope 51 according to the third embodiment, the O-rings 53a and 53b are arranged on the outer circumference of the optical domes 52b and 52c, respectively, and the spaces between the inner circumference of the cylindrical body 2a and the outer circumference of the optical domes 52b and 52c are blocked by the O-rings 53a and 53b at the time of fitting the optical domes 52b and 52c to the cylindrical body 2a. Accordingly, it is not required to apply the filler such as the adhesive to the spaces between the inner circumference of the cylindrical body 2a and the outer circumference of the optical domes 52b and 52c, and the casing 52 with a liquid-tight state being maintained can be easily achieved by fitting the optical domes 52b and 52c to the opening part of the cylindrical body 2a.

As described above, in the capsule medical device according to the third embodiment of the present invention, the O-ring is arranged in the optical dome, and the inner circumference of the cylindrical body is brought into contact with the O-ring over the entire circumference in the circumferential direction of the cylindrical body, at the time of forming the capsule casing by fitting the optical dome to the cylindrical body, thereby closing the space between the inner circumference of the cylindrical body and the outer circumference of the optical dome.
Other configurations of the third embodiment are the same as those of the first embodiment. Accordingly, the same operational effect as those of the first embodiment can be achieved, and the liquid-tight state of the casing can be easily ensured by fitting the optical dome to the opening part of the cylindrical body, without applying the filler to the spaces between the inner circumference of the cylindrical body and the outer circumference of the optical dome.

Further, the work for applying the filler to the space between the inner circumference of the cylindrical body and the outer circumference of the optical dome can be omitted. As a result, the process of manufacturing the capsule medical device can be simplified, and weight saving of the capsule medical device can be facilitated.

In the first to third embodiments and the first and second modifications of the present invention, the adhesive or double-sided tape is applied or attached as a bonding member to the surface of the positioning unit opposed to the optical dome, to fix the illuminating board on the surface of the positioning unit by the bonding member. However, the illuminating board can be fixed to the positioning unit by an adhesive applied to a boundary between a lens frame inserted into and fixed in the through hole of the positioning unit and the illuminating board, the illuminating board can be snap-fitted to the positioning unit, or the illuminating board can be pressed and fixed to the positioning unit by a pressing member screwed to the lens frame inserted into and fixed in the through hole of the positioning unit.

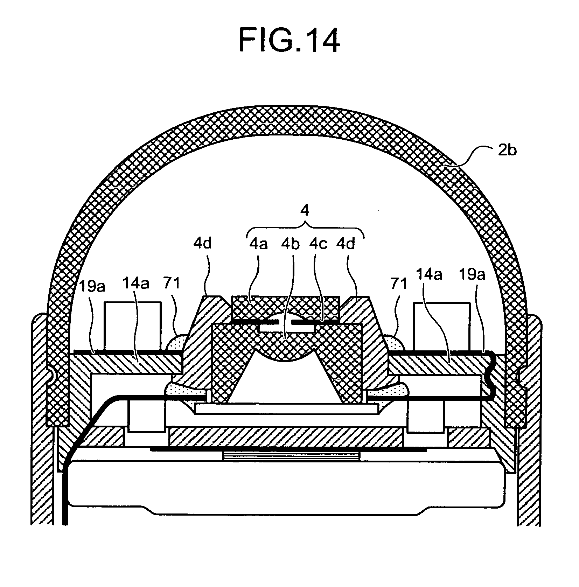

Specifically, in the case of the capsule endoscope 1 according to the first embodiment, as shown in Fig. 14, an adhesive 71 can be applied to a skirt of the lens frame 4d protruding toward the optical dome 2b via the through hole formed on the plate-like portion 14a of the positioning unit 14 to fix the illuminating board 19a on the plate-like portion 14a by the adhesive 71. In this case, because the adhesive 71 is exposed on the illuminating board 19a, a UV-curable adhesive can be used as the adhesive 71. As a result, the illuminating board 19a can be easily fixed to the plate-like portion 14a, as compared with a case of fixing the illuminating board 19a by the bonding member on the plate-like portion 14a.

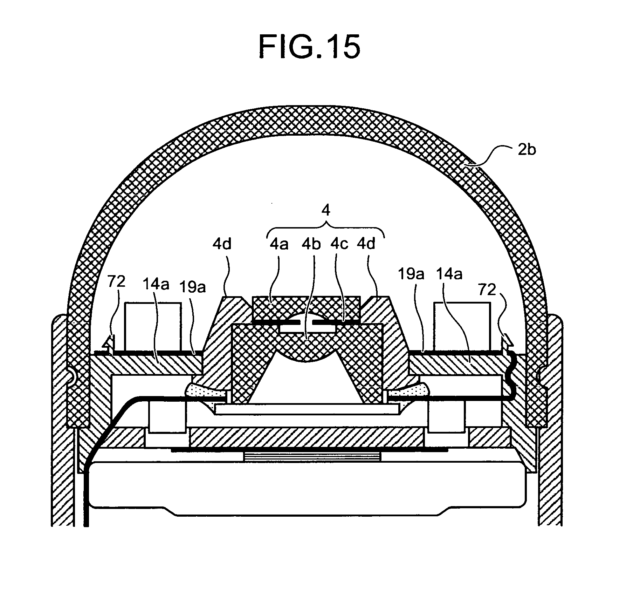

As shown in Fig. 15, one or more hooks 72 that snap-fit the illuminating board 19a can be provided on the surface of the plate-like portion 14a (desirably, on an outer edge of the plate-like portion 14a) opposed to the optical dome 2b to fix the illuminating board 19a on the plate-like portion 14a by snap-fitting using one or more hooks 72. In this case, because it is not required to fix the illuminating board by the bonding member, the illuminating board 19a can be easily fixed to the positioning unit 14.

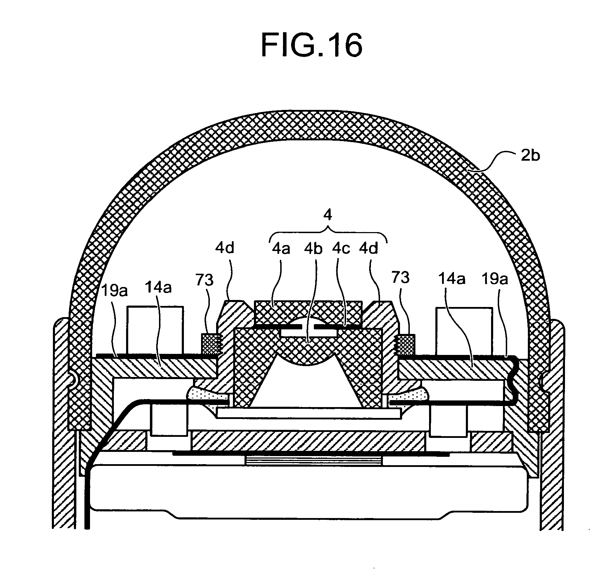

As shown in Fig. 16, a screw portion can be formed near the skirt of the lens frame 4d protruding toward the optical dome 2b via the through hole formed in the plate-like portion 14a of the positioning unit 14, and the illuminating board 19a can be pressed and fixed to the plate-like portion 14a by a pressing force generated by screwing a nut-like pressing unit 73 to the screw portion of the lens frame 4d. In this case, because it is not required to fix the illuminating board by the bonding member, the illuminating board 19a can be easily fixed to the positioning unit 14.

A method of fixing the illuminating board using the adhesive 71, the hook 72, or the pressing unit 73 can be applied to fixation of the illuminating board 19f on the direction B side of the capsule endoscope 1, or can be applied to fixation of the illuminating boards 19a and 19f of the capsule endoscopes 21, 31, 41, and 51 according to the second and third embodiments, or the first and second modifications. When the illuminating board is fixed to the positioning unit, fixation of the illuminating board by the adhesive 71, snap-fitting of the illuminating board by the hook 72, pressing and fixation of the illuminating board by the pressing unit 73, fixation of the illuminating board by the bonding member on the surface of the positioning unit, and fixation of the illuminating board by sandwiching the illuminating board into an engaging portion between the optical dome and the positioning unit can be appropriately combined.

In the second and third embodiments and the second modification of the present invention, the respective lens frames of the positioning unit and the optical unit are formed as separate bodies. However, as exemplified in the first modification, the positioning unit 34 and the lens frame 4d of the optical unit 4 can be integrally formed, and the positioning unit 35 and the lens frame 7d of the optical unit 7 can be integrally formed. In the second and third embodiments, the positioning unit 14 and the lens frame 4d can be integrally formed, and the positioning unit 15 and the lens frame 7d can be integrally formed.

In the first to third embodiments and the first and second modifications of the present invention, as the capsule medical device introduced into the subject, a capsule endoscope having the imaging function and the wireless communication function, which acquires in-vivo images as an example of the in-vivo information is explained. However, the present invention is not limited thereto, and the capsule medical device can be a capsule pH measuring device that measures pH information in a living body as the in-vivo information, a capsule drug-administering device having a function of spraying or injecting a drug into the living body, or a capsule sampling device that samples a substance in the living body (tissue of the body) as the in-vivo information.

In the first to third embodiments and the first and second modifications of the present invention, the twin-lens capsule endoscope including the two solid-state imaging devices 5 and 8 is exemplified, however, the capsule medical device according to the present invention can be a multicular capsule medical device including three or more solid-state imaging devices, or can be a monocular capsule medical device including one solid-state imaging device.

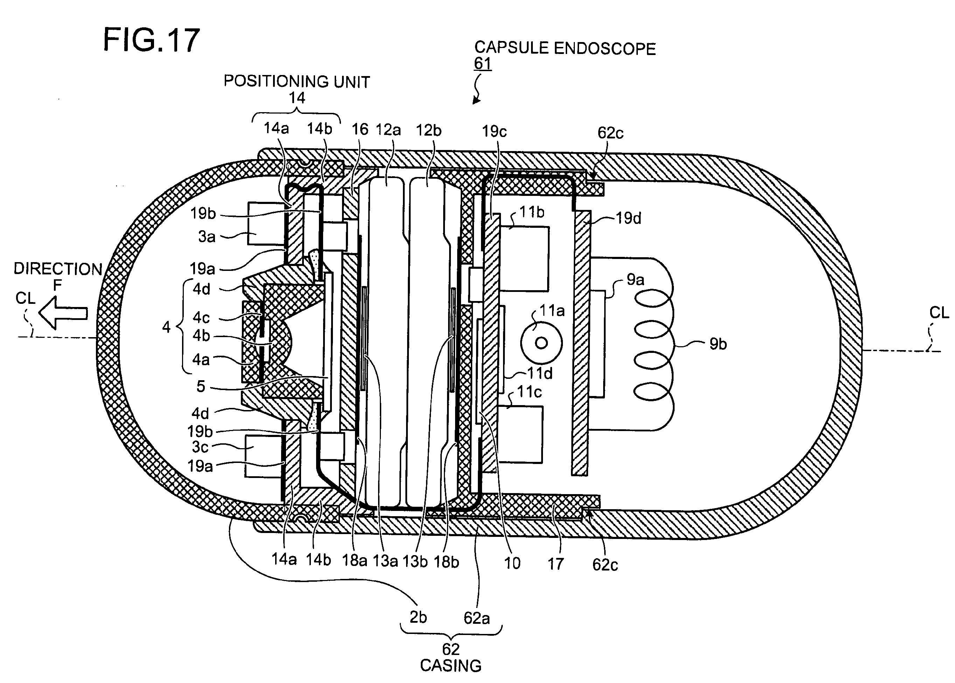

Specifically, as shown in Fig. 17, a monocular capsule endoscope 61 according to the present invention includes a casing 62 instead of the casing 2 of the capsule endoscope 1 according to the first embodiment, and does not include the light-emitting elements 6a to 6d, the optical unit 7, the solid-state imaging device 8, the imaging board 19e, and the illuminating board 19f. In the capsule endoscope 61, the wireless board 19d is a rigid board connected to the control board 19c via a flexible board. The casing 62 is formed by fitting the optical dome 2b to an opening part of a cylindrical body 62a having a dome-like bottom. The load receiving unit 17 fits the end thereof to a step 62c formed on an inner circumference near the bottom of the cylindrical body 62a to fix the position thereof by the elastic force of the contact spring 13b. The load receiving unit 17 ensures the space for arranging the control board 19c and the wireless board 19d and supports the control board 19c and the wireless board 19d without using the filler such as the adhesive to ensure the board interval as in the first embodiment. Other configurations of the third embodiment are the same as those of the first embodiment, and the same reference numerals are given to the same parts. Also in the monocular capsule endoscope 61, the same operational effect as those of the first embodiment can be achieved.

In the first to third embodiments and the first and second modifications of the present invention, the contact spring (the contact springs 13a and 13b) that electrically connects the battery and the power supply board with each other is exemplified as the elastic member that generates the biasing force for pressing and fixing the positioning unit to the end of the optical dome. However, the elastic member can be an exclusive spring that generates the biasing force for pressing the positioning unit to the end of the optical dome via the load receiving unit, which does not electrically connect the battery and the power supply board with each other. In this case, the exclusive spring and the contact springs 13a and 13b are respectively provided at different positions in the casing. The contact springs 13a and 13b are arranged on a support body (a circuit board, battery box, or the like) other than the load receiving unit, together with the batteries 12a and 12b, so that connection between the batteries 12a and 12b and the power supply boards 18a and 18b is not blocked due to the biasing force of the exclusive spring.

Further, in the first to third embodiments and the first and second modifications of the present invention, the biasing force for pressing and fixing the positioning unit to the end of the optical dome is generated by the coil contact springs 13a and 13b. However, the elastic member for generating the biasing force can be an elastic force having a high repulsive force (an elastic member other than the spring) such as urethane. The number of elastic members to be arranged for generating the biasing force is not limited to two like the contact springs 13a and 13b, and can be one or three or more. It is desired to arrange the elastic members such as the contact springs 13a and 13b on the central axis (on the central axis CL) in the longitudinal direction of the capsule casing, or can be respectively arranged at respective positions symmetrical to the central axis.

The spring, which is an example of the elastic member, can be a coil spring as exemplified by the contact springs 13a and 13b, or can be a spring of various shapes such as a plate spring. An external shape of the coil spring can be cylindrical or a cone shape having a long side and a short side. When a cone-shaped contact spring is used instead of the contact springs 13a and 13b, it is desired to connect a long-side end of the cone-shaped contact spring to the power supply board. Accordingly, the biasing force generated by the cone-shaped contact spring can be stably transmitted to the positioning unit.

In the second embodiment of the present invention, the index formed in the optical dome is matched with the upper end of the light-emitting element. However, the index of the optical dome can be matched with a predetermined position of an upper end or a lower end of a component that can be viewable from outside via the optical dome (for example, the light-emitting element, the lens frame of the optical unit, the illuminating board, the positioning unit, or the like) of the built-in unit (functional unit) arranged in the casing of the capsule medical device. Further, the index is formed over the entire circumference in the circumferential direction of the optical dome; however, the index can be formed at least in a part of the optical dome in the circumferential direction, or can be formed in a shape of broken line or dotted line.

In the first to third embodiments and the first and second modifications of the present invention, the positioning unit is pressed to the end of the optical dome by the biasing force of the elastic member exemplified by the contact springs 13a and 13b, thereby fitting and fixing the positioning unit to the end of the optical dome. However, the positioning unit can be press-fit to the inner circumference of the optical dome without using the biasing force of the elastic member, thereby fitting and fixing the positioning unit to the inner circumference of the optical dome. In this case, the internal diameter of the optical dome and the external diameter of the positioning unit can be designed so that a clearance is hardly formed between the inner circumference of the optical dome and the outer circumference of the positioning unit.

Further, the capsule medical device according to the present invention can be formed by appropriately combining the first to third embodiments and the first and second modifications. For example, the indexes M1 and M2 or the O-rings 53a and 53b can be formed in the optical domes 2b and 2c of the capsule endoscopes 1 and 21 according to the first embodiment or the first modification, or these can be combined. Similarly, the indexes M1 and M2 or the O-rings 53a and 53b can be provided in the optical domes 32b and 32c of the capsule endoscope 31 according to the second modification of the first embodiment, or these can be combined. Further, the O-rings 53a and 53b can be provided on the optical domes 42b and 42c of the capsule endoscope 41 according to the second embodiment, or the indexes M1 and M2 can be formed in the optical domes 52b and 52c of the capsule endoscope 51 according to the third embodiment.

In the first to third embodiments and the first and second modifications of the present invention, the biasing force of the elastic member exemplified by the contact springs 13a and 13b is transmitted to the positioning unit via the load receiving unit; however, the biasing force of the elastic member can be directly transmitted to the positioning unit, without using the load receiving unit.

Further, in the third embodiment of the present invention, the O-rings 53a and 53b are arranged on the outer circumference of the optical domes 52b and 52c; however, the O-rings 53a and 53b can be arranged at a part on the inner circumference of the cylindrical body 2a and coming into contact with the outer circumference of the optical domes 52b and 52c.

### INDUSTRIAL APPLICABILITY

As described above, the capsule medical device and the method of manufacturing the capsule medical device according to the present invention are useful for a capsule medical device introduced into a subject, and particularly suitable for a capsule medical device that can suppress variations in a relative positional relation of an optical dome, an illuminating unit, and an optical unit of a capsule casing, and can determine the respective preferable relative positions of the illuminating unit and the optical unit with respect to the optical dome highly accurately, and a method of manufacturing the capsule medical device.

## Claims

1. A capsule medical device comprising:
an optically transparent optical dome forming a part of a capsule casing introduced into a subject;
an illuminating board having an illuminating unit that illuminates inside the subject over the optical dome fixedly arranged thereon;
an optical unit that forms an image of inside the subject illuminated by the illuminating unit;
an imaging unit that is fixedly arranged with respect to the optical unit and captures images of inside the subject; and
a positioning unit in which the illuminating board and the optical unit are fixedly arranged, which is fitted and fixed to an inner circumference of the optical dome to determine relative positions of the illuminating board and the optical unit with respect to the optical dome.

2. The capsule medical device according to claim 1, wherein
the positioning unit comprises:
a plate-like portion having the illuminating board and the optical unit fixedly arranged thereon, and having an outer circumference fitted to an inner circumference of the optical dome; and
a protrusion protruding from the plate-like portion for fixing the plate-like portion to the inner circumference of the optical dome by being engaged with an opening end of the optical dome.

3. The capsule medical device according to claim 1, wherein
the positioning unit is a plate-like portion having the illuminating board and the optical unit fixedly arranged thereon, and having an outer circumference fitted to an inner circumference of the optical dome, and
the optical dome has a step for connecting a first inner circumference to be fitted to the outer circumference of the plate-like portion with a second inner circumference having an internal diameter smaller than that of the first inner circumference, so that an outer edge of the plate-like portion is engaged with the step, thereby fixing the plate-like portion to the inner circumference of the optical dome.

4. The capsule medical device according to claim 2 or 3, wherein
the optical unit comprises:
one or more lenses that form an image of inside the subject onto a light receiving surface of the imaging unit; and
a lens frame that holds the one or more lenses, and wherein
the lens frame is inserted into and fixed in a through hole formed in the plate-like portion.

5. The capsule medical device according to claim 2 or 3, wherein
the optical unit includes one or more lenses that form an image of inside the subject on a light receiving surface of the imaging unit, and
the plate-like portion integrally includes a lens frame that holds the one or more lenses.

6. The capsule medical device according to claim 4, wherein
the lens frame includes an abutment portion protruding in a radial direction of the lens frame, which forms an external diameter larger than an internal diameter of the through hole formed in the plate-like portion, and
the illuminating board has an opening part into which the lens frame is inserted, and is fixedly arranged on a surface of the plate-like portion opposed to the optical dome, in a state with the lens frame being inserted into the opening part and the plate-like portion being abutted to the abutment portion.

7. The capsule medical device according to claim 5, wherein the illuminating board has an opening part into which the lens frame is inserted, and is fixedly arranged on a surface of the plate-like portion opposed to the optical dome, in a state with the lens frame being inserted into the opening part.

8. The capsule medical device according to claim 6 or 7, wherein the illuminating board is fixed by a bonding member on the surface of the plate-like portion.

9. The capsule medical device according to claim 6 or 7, wherein the illuminating board is fixed to the lens frame with an adhesive applied to a skirt of the lens frame protruding via the opening part.

10. The capsule medical device according to claim 6 or 7, wherein
the plate-like portion comprises one or more hooks that snap-fit the illuminating board on the surface opposed to the optical dome, and
the illuminating board is fixed to the plate-like portion by snap-fitting by the one or more hooks.

11. The capsule medical device according to claim 6 or 7, further comprising a pressing unit that is screwed to a skirt of the lens frame protruding via the opening part, and presses the illuminating board to the plate-like portion, wherein
the illuminating board is fixed to the lens frame by a pressing force of the pressing unit.

12. The capsule medical device according to any one of claims 4 to 11, wherein the positioning unit determines a relative position of the illuminating unit with respect to the optical dome in a state with an upper end of the illuminating unit being positioned at a position lower than an upper end of the lens frame.

13. The capsule medical device according to any one of claims 1 to 12, wherein the illuminating board is a flexible circuit board.

14. The capsule medical device according to any one of claims 1 to 13, wherein the positioning unit further determines a relative position of the imaging unit with respect to the optical dome.

15. The capsule medical device according to any one of claims 1 to 14, further comprising an antenna that transmits a wireless signal including an image inside the subject captured by the imaging unit to outside, wherein
the positioning unit further determines a relative position of the antenna with respect to the optical dome.

16. The capsule medical device according to claim 1, further comprising:
an elastic member that generates a biasing force for biasing the positioning unit in a direction of pressing the positioning unit toward the optical dome; and
a load receiving unit that receives the biasing force and transmits the biasing force to the positioning unit, wherein
the positioning unit is fitted and fixed to the inner circumference of the optical dome by the biasing force of the elastic member transmitted via the load receiving unit.

17. The capsule medical device according to claim 16, further comprising a power supply unit that supplies power to at least the imaging unit, wherein
the elastic member is a contact spring that ensures a contact point of the power supply unit.

18. The capsule medical device according to claim 16 or 17, wherein the optical dome has a contact surface with which at least a part of the positioning unit comes into contact.

19. The capsule medical device according to any one of claims 16 to 18, wherein the positioning unit is a resin member formed by resin molding.

20. The capsule medical device according to any one of claims 16 to 19, wherein
the imaging unit includes a plurality of imaging units that captures images of inside the subject in a direction different from each other,
the capsule casing includes a plurality of the optical domes corresponding to the plurality of the imaging units,
a plurality of sets, corresponding to the plurality of the imaging units, of the illuminating unit, the illuminating board, the optical unit, the positioning unit, and the load receiving unit are incorporated in the capsule casing, and
the biasing force of the elastic member acts in a direction opposing to each of the imaging units.

21. The capsule medical device according to any one of claims 16 to 20, wherein an expansion and contraction distance of the elastic member is larger than a size variation of a built-in unit of the capsule casing in an acting direction of the biasing force.

22. The capsule medical device according to any one of claims 16 to 21, wherein
the optical dome is fitted to an end of a cylindrical body forming a remaining part of the capsule casing by engagement of protruding and depressed portions, and
the biasing force of the elastic member is smaller than a retaining force of the optical dome and the cylindrical body by the engagement of protruding and depressed portions.

23. The capsule medical device according to claim 22, wherein an external diameter of the built-in unit of the capsule casing is smaller than an internal diameter of the cylindrical body.

24. The capsule medical device according to claim 22 or 23, further comprising an O-ring that closes a space on a connecting surface between the optical dome and the cylindrical body.

25. The capsule medical device according to any one of claims 16 to 24, wherein the optical dome is provided with an index with a state that a relative position with respect to the built-in unit of the capsule casing can be viewed from outside.

26. A capsule medical device comprising:
an optically transparent optical dome forming a part of a capsule casing introduced into a subject;
an illuminating board having an illuminating unit that illuminates inside the subject over the optical dome fixedly arranged thereon;
an optical unit that forms an image of inside the subject illuminated by the illuminating unit;
an imaging unit that is fixedly arranged with respect to the optical unit and captures images of inside the subject;
a positioning unit in which the illuminating board and the optical unit are fixedly arranged, which is fitted and fixed to an inner circumference of the optical dome to determine relative positions of the illuminating board and the optical unit with respect to the optical dome;
an elastic member that generates a biasing force for biasing the positioning unit in a direction of pressing the positioning unit toward the optical dome; and
a load receiving unit that receives the biasing force and transmits the biasing force to the positioning unit, wherein
the positioning unit is fitted and fixed to the inner circumference of the optical dome by the biasing force of the elastic member transmitted via the load receiving unit.

27. A capsule medical device comprising:
an optically transparent optical dome forming a part of a capsule casing introduced into a subject;
an illuminating board having an illuminating unit that illuminates inside the subject over the optical dome fixedly arranged thereon;
an optical unit that forms an image of inside the subject illuminated by the illuminating unit;
an imaging unit that is fixedly arranged with respect to the optical unit and captures images of inside the subject;
a positioning unit in which the illuminating board and the optical unit are fixedly arranged, which is fitted and fixed to an inner circumference of the optical dome to determine relative positions of the illuminating board and the optical unit with respect to the optical dome;
an elastic member that generates a biasing force for biasing the positioning unit in a direction of pressing the positioning unit toward the optical dome;
a control unit that controls an operation of the imaging unit; and
a support body that supports a circuit board having the control unit mounted thereon and ensures board spacing between the circuit board and another board, wherein
the support body is brought into contact with and fitted to the positioning unit to transmit the biasing force of the elastic member, thereby pressing the positioning unit to the optical dome.

28. A method of manufacturing a capsule medical device, comprising:
a step of fixedly arranging an optical unit for forming light on an imaging unit in the imaging unit;
a step of inserting the optical unit into a through hole formed in a positioning unit to fix the optical unit therein;
a step of inserting the optical unit into a through hole formed in an illuminating board having an illuminating unit to fix the optical unit in the positioning unit; and
a step of determining respective relative positions of the optical unit, the illuminating board, and the optical dome by fitting and fixing the positioning unit to an inner circumference of the optical dome forming a part of a capsule casing.

29. The method of manufacturing a capsule medical device according to claim 28, further comprising a step of maintaining the respective relative positions of the optical unit, the illuminating board, and the optical dome by pressing the positioning unit to the optical dome by a biasing force generated by an elastic member.
